# EUROPEAN PATENT SPECIFICATION

(11) **EP 4 087 853 B1**
(45) Date of publication and mention of the grant of the patent: **18.10.2023**
(21) Application number: 21700012.4
(22) Date of filing: 08.01.2021
(51) Int. Cl.: C07H 15/04, A61P 35/00

(54) **NEW IMMUNOSTIMULATORS AND USE THEREOF IN IMMUNOTHERAPY**
NEUE IMMUNSTIMULATOREN UND VERWENDUNG DAVON IN DER IMMUNTHERAPIE
NOUVEAUX IMMUNOSTIMULATEURS ET LEUR UTILISATION EN IMMUNOTHÉRAPIE

(30) Priority: 10.01.2020 EP 20305019
(43) Date of publication of application: 16.11.2022
(73) Proprietor: NANTES UNIVERSITÉ, 44000 Nantes (FR); Centre national de la recherche scientifique, 75016 Paris (FR); INSTITUT NATIONAL DE LA SANTE ET DE LA RECHERCHE MEDICALE - INSERM, 75013 Paris (FR)
(72) Inventor: DUBREUIL, Didier, 44000 NANTES (FR); PATINEC, Allan, 44000 NANTES (FR); PIPELIER, Muriel, 44300 NANTES (FR); LE PENDU, Jacques, 44000 NANTES (FR); LEBRETON, Jacques, 44300 NANTES (FR); BLOT, Virginie, 44980 SAINTE-LUCE-SUR-LOIRE (FR); TESSIER, Arnaud, 44700 ORVAULT (FR)
(74) Representative: Lavoix
(86) International application number: PCT/EP2021/050276
(87) International publication number: WO 2021/140203

(56) References cited:
- EP-A1- 2 842 961
- WO-A1-2014/001204
- PETER J JERVIS ET AL: "New CD1d agonists: Synthesis and biological activity of 6-triazole-substituted -galactosyl ceramides", BIOORGANIC & MEDICINAL CHEMISTRY LETTERS, PERGAMON, AMSTERDAM, NL, vol. 22, no. 13, 2 May 2012 (2012-05-02), pages 4348-4352, XP028491007, ISSN: 0960-894X, DOI: 10.1016/J.BMCL.2012.05.009 [retrieved on 2012-05-09]
- NORA PAUWELS ET AL: "Synthesis of 6''-triazole-substituted [alpha]-GalCer analogues as potent iNKT cell stimulating ligands", BIOORGANIC & MEDICINAL CHEMISTRY, vol. 20, no. 24, 1 December 2012 (2012-12-01), pages 7149-7154, XP055057201, ISSN: 0968-0896, DOI: 10.1016/j.bmc.2012.09.063

## Description

### Field of the invention

The present invention relates to immunostimulators and the use thereof in immunotherapy.

### Technical background

iNKT (*Invariant Natural Killer T*) cells are a unique population of lymphocytes able to specifically recognize glycolipid antigens. These antigens need to be presented through a non-classical MHC-I (*Major Histocompatibility Complex class I*) molecule, CD1d, for example by antigen presenting cells.

KRN7000 (also called α-GalCer or alpha-Galactosylceramide) is a canonical ligand of iNKT cells. KRN7000 is in fact a synthetic glycolipid derived from agelasphin 9b produced by the marine sponge *Agelas mauritianus.* In response to the binding of KRN7000 to iNKT cells, these cells rapidly release copious quantities of cytokines able to initiate or amplify an immune response. KRN7000 was shown to protect against LPS-induced shock and display potent antitumor activity in various *in vivo* models. KNR7000-pulsed DCs (*Dendritic Cells*) or loaded on CD1d receptor was thus studied in clinical trials, such as for the treatment of chronic hepatitis B and C infections and cancer therapy (in particular non-small cell cancer, lung cancer, metastatic carcinoma, liver, breast, neck skin, prostate and head cancers, melanoma and solid tumors (myelodysplastic syndromes, multiple myeloma, ).

KRN7000 has the following formula (I):

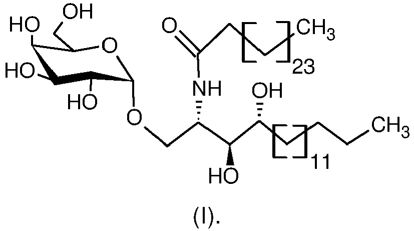

Number of analogues of KNR7000 have been disclosed, including few 6"-O-modified GalCer derivatives, but the latter series of analogues presented the same or a slight improvement (30-40%) of iNKT activation by comparison with the reference KRN7000, with some time better Th1 bias.

Further iNKT modulators are disclosed in WO 2014/001204 A1, EP 2 842 961 A1, P. J. JERVIS et al.: BIOORGANIC & MEDICINAL CHEMISTRY LETTERS, 2012 vol. 22, , pp 4348-4352 and N. PAUWELS et al.: BIOORGANIC & MEDICINAL CHEMISTRY, 2012, vol. 20, no. 24, pp. 7149-7154.

There is still a need to provide new immunostimulators, which are preferably more efficient and/or preferably have a better targeting therapeutic selectivity for use in immunotherapy, in particular for the prevention and/or treatment of cancer.

### Detailed description

The Inventors have synthetized a new family of stimulators of human iNKT cells, which stimulators were surprisingly found to be the most powerful iNKT stimulators known to date, for some of them much higher than the reference glycolipid KRN7000.

The iNKT stimulators of this new family comprise or consist of a compound of the following formula (II): 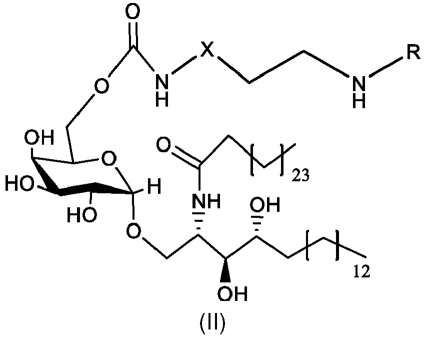
wherein X represents a PEG fragment -[CH₂-CH₂-O]ₘ- with m being an integer from 1 to 24, an alkyl chain -(CH₂)ₙ- with n being an integer from 1 to 24 or a branched hydrocarbon group,
wherein R represents H, -CO-R1, -CONR1, -COOR1, -CSR1, -CSNR1, or - CSOR1,
wherein R1 represents an alkyl, an aryl, an heterocyclic group, a PEG fragment - [CH₂-CH₂-O]ₚ-R2 with p being an integer from 1 to 24 or an alkyl chain -(CH₂)_{q}-R2 with q being an integer from 1 to 24,
wherein R2 represents a functional reacting group allowing coupling to a carrier D or represents L-D, wherein L is a linker and D is a carrier.

For example, compound 6-PEG₃-NH₂-GalCer 2a of the following formula (IV): 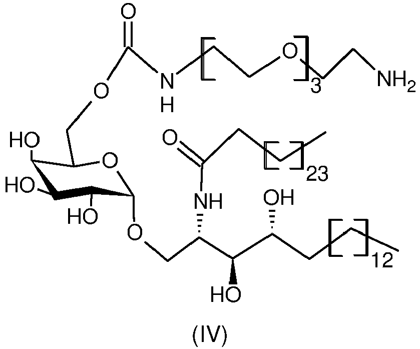 displays a potency nearly 10³ fold higher than those of parent KRN7000 on cytokine release from iNKT, while the 6-PEG₃-NHAc-GalCer 2b counterpart of the following formula (V): 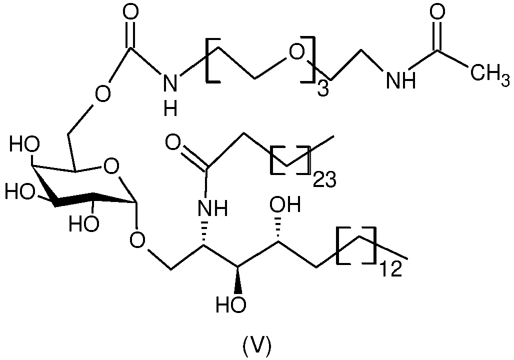 shows a potency 10⁴⁻⁵ fold higher.

Because of their potent biological activity, these iNKT stimulators disclosed herein are very useful in the context of immunotherapy, in particular against cancer and other diseases, such as bacterial infections, viral infections, parasitic diseases or autoimmune diseases (for example type I diabetes or multiple sclerosis).

Furthermore, another advantage of the iNKT stimulators disclosed herein is their unexpected ability to use tumor cells, which were so far considered as "non-CD1d cells", as presenting cells themselves, to activate iNKTs without requiring the mobilisation of canonical APCs (*Antigen Presenting Cells*), such as DCs (*Dendritic Cells*), macrophages, *etc..*

Among the iNKT stimulators disclosed herein is 6-Mal-PEG₆GalCer 3a (derived from 6-PEG₃-NHR-GalCer 2a of formula (IV)) and having the following formula (VII):

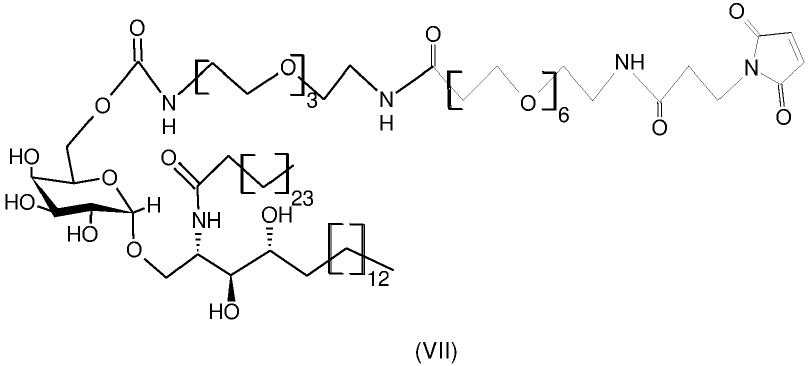

The iNKT stimulator of formula (VII) displays a INKT activation potency nearly 10 fold higher than those of parent KRN7000 and can advantageously be linked to a biological carrier, such as a protein or an antibody, or be vectorized, for example in nanoparticles, such as virus-like particles or liposomes. This coupling may advantageously allow delivering the iNKT stimulator to the target cells, thereby reducing the therapeutic dose needed to reach the therapeutic effect and/or reducing the possible side effects.

For example, Cetuximab is a humanized monoclonal antibody targeting EGFR (*Epidermal Growth Factor Receptor*); the coupling of cetuximab antibody to an iNKT stimulator as disclosed herein allows inducing the known immunological effect (ADCC (*Antibody-Dependent Cell-mediated Cytotoxicity*)) of said antibody concomitantly to an activation of iNKT cells, thereby releasing cytokines. A bioconjugate comprising cetuximab antibody coupled to an iNKT stimulator therefore results in combined biological effects on tumor targets, by reactivating the immune system close to the tumor environment.

The bioconjugate comprising an antibody coupled to an iNKT stimulator is preferably a non-enzymatic cleavable linked glycoconjugate, so as to lead to a great selectivity at inducing iNKT stimulation in the tumor environment, without being effective elsewhere in the body (or minorly). Consequently, the bioconjugate comprising an antibody coupled to an iNKT stimulator described above allows reducing or suppressing several side effects induced by non-located activation of iNKT by the immunostimulator alone and achieve selective tumor targeting.

A first objet of the invention is thus an iNKT stimulator, wherein said iNKT stimulator comprises a compound of the following formula (II): 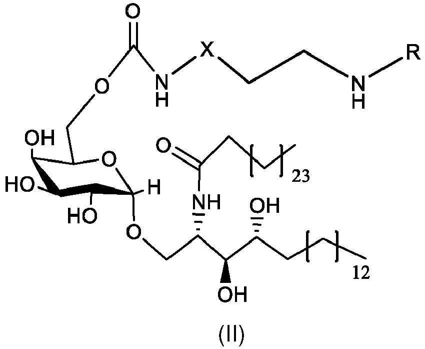
wherein X represents a PEG fragment -[CH₂-CH₂-O]ₘ with m being an integer from 1 to 24, an alkyl chain -(CH₂)ₙ- with n being an integer from 1 to 24 or a branched hydrocarbon group,
wherein R represents -CO-R1, -CONR1, -COOR1, -CSR1, -CSNR1 or -CSOR1, wherein R1 represents an alkyl, an aryl, an heterocyclic group, a PEG fragment - [CH₂-CH₂-O]ₚ-R2 with p being an integer from 1 to 24 or an alkyl chain -(CH₂)_{q}-R2 with q being an integer from 1 to 24,
wherein R2 represents a functional reacting group allowing coupling to a carrier D or represents L-D, wherein L is a linker and D is a carrier.

The iNKT stimulator as defined above is preferably selected from the group consisting of:
- a compound of the following formula (V): 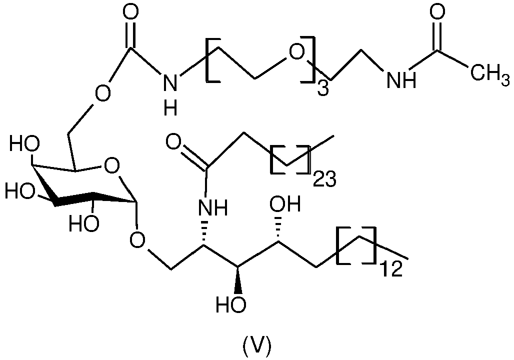
- a compound of the following formula (VI): 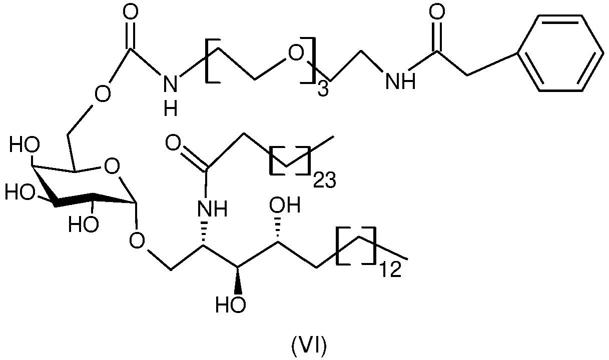
- a compound of the following formula (VII): 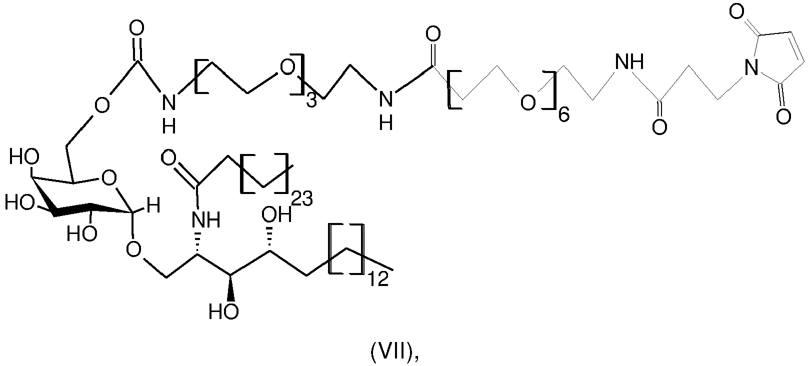 and
- a compound of the following formula (VIII):

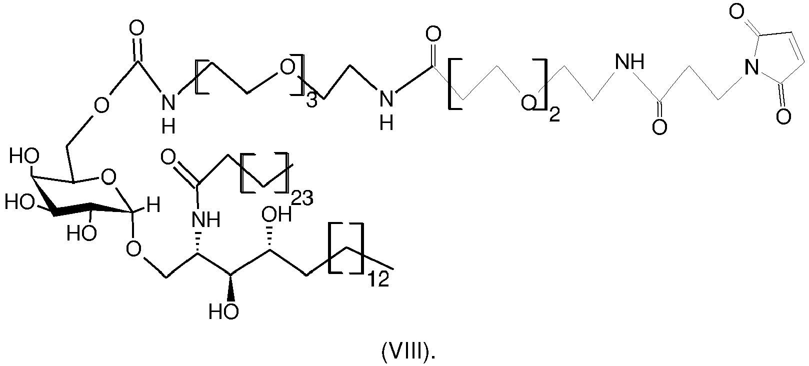

Another object of the invention is a conjugate comprising at least one iNKT stimulator coupled to at least one carrier, wherein said conjugate:
(i) comprises a compound of the formula (II) as defined above wherein X represents a PEG fragment -[CH₂-CH₂-O]ₘ with m being an integer from 1 to 24, an alkyl chain -(CH2)ₙ- with n being an integer from 1 to 24 or a branched hydrocarbon group, wherein R represents -CO-R1, -CONR1, -COOR1, -CSR1, -CSNR1 or -CSOR1 and wherein R1 represents a PEG fragment -[CH₂-CH₂-O]ₚ-L-D with p being an integer from 1 to 24 or an alkyl chain -(CH₂)_{q}-L-D with q being an integer from 1 to 24, wherein L is a linker and D is a carrier, or
(ii) is obtained by coupling to a carrier D an iNKT stimulator of the formula (II) as defined above, wherein X represents a PEG fragment -[CH₂-CH₂-O]ₘ with m being an integer from 1 to 24, an alkyl chain -(CH2)ₙ- with n being an integer from 1 to 24 or a branched hydrocarbon group, wherein R represents -CO-R1, - CONR1, -COOR1, -CSR1, -CSNR1 or -CSOR1, wherein R1 represents a PEG fragment -[CH2-CH2-O]ₚ-R2 with p being an integer from 1 to 24 or an alkyl chain -(CH₂)_{q}-R2 with q being an integer from 1 to 24 and wherein R2 represents a functional reacting group allowing coupling to said carrier D.

In the iNKT stimulator as defined above or in the conjugate as defined above, the carrier D is preferably a therapeutic agent and/or a targeting agent.

The carriers D as defined above may be an antibody, antibody fragment, sugar, lectin, affitin, growth factor, antigen, a chemical molecule, a protein, peptide, glycoprotein, aptamer, loaded cell, virus and/or carbohydrate.

Another object of the invention is a vector, for example a nanoparticle, comprising at least one iNKT stimulator as defined above, at least one conjugate as defined above and/or at least one iNKT stimulator comprising a compound of the following formula (IV):

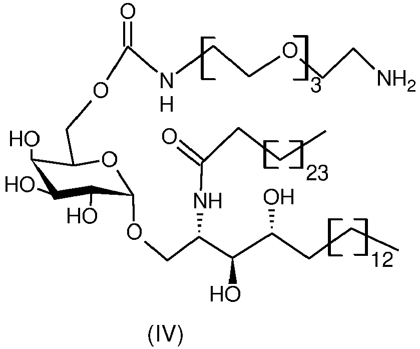

Another object of the invention is a pharmaceutical composition comprising:
(i) at least one iNKT stimulator as defined above, at least one conjugate as defined above, at least one vector as defined above and/or at least one iNKT stimulator comprising a compound of the following formula (IV) as defined above, and
(ii) at least one pharmaceutically acceptable vehicle.

Another object of the invention relates to the iNKT stimulator as defined above, the conjugate as defined above, the vector as defined above, the pharmaceutical composition as defined above or an iNKT stimulator comprising a compound of the formula (IV) as defined above, for use as a medicament, preferably in the prevention and/or treatment of cancer, an autoimmune disease, an inflammatory disease, a viral infection, a bacterial infection and/or a parasitic disease.

Another object of the invention relates to the *in vitro* use of the iNKT stimulator as defined above, of the conjugate as defined above, of the vector as defined above or of an iNKT stimulator comprising a compound of the following formula (IV) as defined above to activate iNKT cells.

### iNKT stimulator

The present invention thus provides a new family of iNKT stimulators.

By the expression "iNKT stimulator", also referred to as "iNKT activator", it is herein meant an immunostimulator of iNKT cells, i.e. a compound able to activate iNKT cells.

By the expression "a compound able to activate iNKT cells", it is herein meant that, in the presence of said compound and of a CD1d expressing cell, iNKT cells express or increase the expression of at least one cytokine by comparison to in the absence of said compound.

The at least one cytokine whose expression is induced or increased in the presence of an iNKT stimulator a Th1 or Th2 cytokine (preferably a Th1 cytokine for cancer therapy), such as for example a cytokine selected from the group consisting of interferon gamma (INF-y), TNF-α and interleukin (such as IL-13, IL-2, IL-10, IL12 or IL4).

CD1d is a non-polymorphic major histocompatibility complex class I-like antigen presenting molecule.

The CD1d expressing cell, also referred to as "CD1d cell", may be a cell naturally expressing CD1d or a cell transfected to express CD1d.

The CD1d expressing cell may be a cell expressing CD1d at low level or high level.

By "cell expressing CD1d at low level", it is herein meant that CD1d expressed by this cell is not detected by flow cytometry but can be detected by RT-PCR. Cells expressing CD1d at low level were considered until now as "non-CD1d-Cells".

By "cell expressing CD1d at high level", it is herein meant that CD1d expressed by this cell can be detected by RT-PCR and flow cytometry.

A cell expressing CD1d at low level is for example a tumoral cell, such as HeLa cells (i.e. non transfected HeLa cells), HEK293 cells (i.e. non transfected HEK293 cells), Meso13 cells, Meso34 cells, Meso225 cells (from breath cancer), SW116 cells or HTC116 cells (from colorectal cancer).

Non-limitative examples of cells expressing CD1d at high level are APCs (*Antigen Presenting cells*), such as monocytes, macrophages, dendritic cells, B lymphocytes, epithelial cells (in particular from colon or intestine) and any cells transfected to express CD1d.

Cells expressing CD1d at high level may be provided in the form of PBMCs (*Peripheral Blood Mononuclear Cells*). PBMCs may be obtained by any method well known by the skilled person, such as extraction from whole blood using Ficoll by density gradient centrifugation.

iNKT cells (*Invariant Natural Killer T cells*) are a population of lymphocytes recognizing CD1d. INKT cells (Invariant Natural Killer T cells) express markers from NK cells (such as CD16 and CD56) and from T cells (such as CD3, CD4 and CD8). Their main particularity is the expression of an invariant TCR restricted to the CD1d molecule. This TCR only recognizes lipids and glycolips antigen and not peptide like classical TCR.

The best-known natural antigen of iNKT cells is KRN7000.

To assess if a compound is an iNKT stimulator, any method well known by the skilled person may be used.

Classically, CD1d expressing cells are incubated overnight in presence or in the absence of the compound to be assessed, allowing loading on the CD1d molecule. Then, CD1d cells are co-incubated with iNKTs. Six hours of co-culture are sufficient to be able to detect most of the cytokine release (such as IFN-γ, IL-13 and/or IL-2) in the supernatant. Various time of incubation, up to 48H, can be performed depending of the kinetic of some late cytokines (such as IL-10). The quantity or concentration of at least one cytokine is thus measured in the supernatant after an incubation time of from 6 hours to 48 hours. An increased quantity or concentration of at least one cytokine in the presence of the assessed compound by comparison to in its absence means that said compound is a iKNT stimulator.

When cells expressing CD1d at low level are used in the above assay, an increased quantity or concentration of at least one cytokine in the presence of said compound by comparison to in its absence means that this compound is a potent iKNT stimulator.

### 6-PEGm-NHR-GalCer compounds

The iNKT stimulator preferably comprises or consists of a KRN7000 analogue of the following formula (II), also referred to herein as compound 6-PEGm-NHR-GalCer: 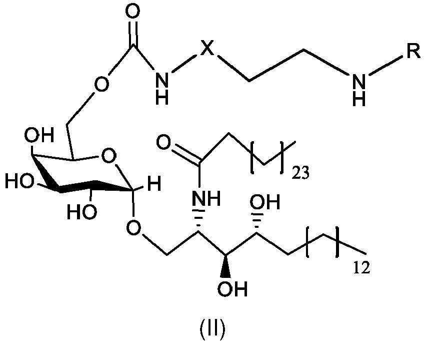
wherein X represents a PEG fragment -[CH₂-CH₂-O]ₘ- with m being an integer from 1 to 24, an alkyl chain -(CH₂)ₙ- with n being an integer from 1 to 24 or a branched hydrocarbon group,
wherein R represents H, -CO-R1, -CONR1, -COOR1, -CSR1, -CSNR1, or -CSOR1, wherein R1 represents an alkyl, an aryl, an heterocyclic group, a PEG fragment -[CH₂-CH₂-O]ₚ-R2 with p being an integer from 1 to 24 or an alkyl chain -(CH₂)_{q}-R2 with q being an integer from 1 to 24,
wherein R2 represents a functional reacting group allowing coupling to a carrier D or represents L-D, wherein L is a linker and D is a carrier.

When R2 represents a functional reacting group allowing coupling to a carrier D, R2 is for example N-maleimide, N₃, alkyne, alkene, acryloyl, norbornene, cyclooctadiene, TCO, tetrazine, isocyanate, thiol or aldehyde.

The functional reacting group may allow coupling to a carrier by various ways, such as through lysines, reductive-coupling to oxidized carbohydrates,through cysteine residues and/or through hydrazone-, disulfide- and/or peptide-based linkages.

A variety of linkage heterobifunctional reagents between Ligand and Drug conjugate have been described in the literature and may be used for coupling the iNKT stimulator to the carrier via the functional reacting group.

The carrier D is for example as defined below in the section "carrier".

The linker is preferably a covalent linker.

The linker, in particular the covalent linker, is preferably a non enzymatic cleavable linker.

The linker for example originates from the method for coupling to the carrier the compound of formula (II) as defined above, wherein R1 represents a PEG fragment -[CH₂-CH₂-O]ₚ-R2 with p being an integer from 1 to 24 or an alkyl chain -(CH₂)_{q}-R2 with q being an integer from 1 to 24 and wherein R2 represents a functional reacting group allowing coupling to a carrier D.

The linker for example comprises or consists of the compound of the following formula (XI): 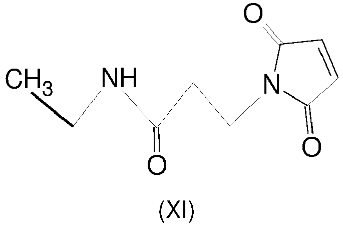 or the following formula (XIII):

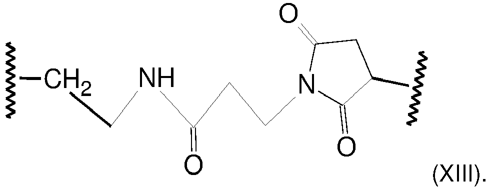

In one embodiment, the iNKT stimulator as defined above is characterized in that R has the the following formula (III): 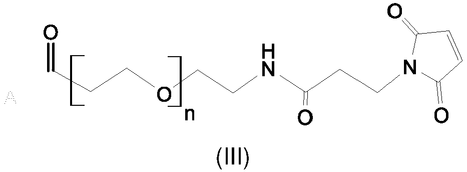 or the following formula (XIV):

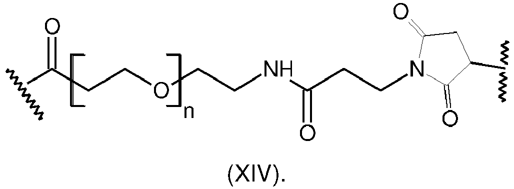

The iNKT stimulator as defined above wherein R is of formula (III) may result from the reaction of the iNKT stimulator of formula (II), in particular wherein R represents H, with a SM(PEG)n crosslinker.

By "SM(PEG)n linker", it is herein meant a Succinimide-PEGₙ-Maleimide linker.

In the PEG fragment -[CH₂-CH₂-O]ₘ, m is an integer from 1 to 24, preferably from 1 to 20, preferably from 1 to 15, more preferably from 1 to 12. For example, m may be 1, 2, 3, 4, 5, 6, 7, 8, 9, 10 or 12, preferably 1, 3, 6 or 12.

In the PEG fragment -[CH₂-CH₂-O]ₚ-R2, p is an integer from 1 to 24, preferably from 1 to 20, preferably from 1 to 15, more preferably from 1 to 10. For example, p may be 1, 2, 3, 4, 5, 6, 7, 8, 9, 10, 11 or 12, preferably 1, 3, 6 or 12.

By "alkyl", it is herein meant a linear hydrocarbon group comprising from 1 to 24 carbon atoms, preferably from 1 to 12 carbon atoms, or a branched or cyclic hydrocarbon group comprising from 1 to 12 carbon atoms. Examples of alkyl groups include methyl, ethyl, n-propyl, isopropyl, n-butyl, isobutyl, tert-butyl, n-pentyl, isopentyl, n-hexyl, and cyclohexyl groups, preferably methyl or ethyl.

In the alkyl chain -(CH₂)ₙ-, n is preferably an integer from 1 to 24, for example from 1 to 20, from 1 to 15, from 1 to 12, from 1 to 10. For example, n may be 1, 2, 3, 4, 5, 7, 8, 9, 10, 11 or 12, preferably 1, 3, 6 or 12.

In the alkyl chain -(CH₂)_{q}-R2, q is preferably an integer from 1 to 24, for example from 1 to 20, from 1 to 15, from 1 to 10,. For example, q may be 1, 2, 3, 4, 5, 7, 8, 9, 10, 11 or 12, preferably 1, 3, 6 or 12.

By "branched hydrocarbon group", it is herein meant a branched hydrocarbon chain having 1, 2, 3, 4, 5, 6, 7, 8, 9, 10, 11 or 12 carbon atoms, whose chain may be linear or ramified, saturated or may comprise one or more double or triple bonds and whose chain may be interrupted by one or more heteroatoms or substituted by one or more heteroatoms or by one or more substituents comprising a heteroatom. In this respect, by "heteroatom", it is herein meant any atom other than carbon or hydrogen, this atom typically being a nitrogen, oxygen or sulphur atom.

By "aryl", it is herein meant a monocyclic or polycyclic aromatic hydrocarbon group, which may be optionally substituted by acid, amide, amide, urea, amine, thiol, alcohol, ether, ester groups. The aryl is preferably a phenyl, benzyl, napththyl, anthracenyl. The aryl may be substituted by at least one alkyl group. A preferred example of aryl is phenyl group.

By "heterocyclic group", it is herein meant a unsubstituted or substituted group that contains as constituent elements of the ring 1 to 4 heteroatoms selected from the group consisting of a nitrogen atom, oxygen atom and sulfur atom. The heterocyclic group may be monocyclic or polycyclic.

Examples of heterocyclic groups include 3-membered rings, 4-membered rings, 5-membered rings, 6-membered rings, 7-membered rings, 8-membered rings and 9-membered rings.

Examples of 5-membered rings include pyrrolidine, pyrrole, tetrahydrofuran, furan, tetrahydrothiophene, thiophene, imidazoline, imidazole, pyrazolidine, pyrazole, oxazolidine, oxasole, isoxazolidine, isoxasole, dioxolane, tithiolane.

Examples of 6-membered rings include piperidine, pyridine, tetrahydropyran, pyran, thiane, thiopyran, piperazine, diazine, morpholine, oxazine, thiomorpholine, thiazine, dioxane, dioxine, dithiane, dithiin, hexahydro-1,3,5-triazine, triazine, trioxane, trithiane, tetrazine, pentazine, triazole, furazan, oxadiazole, thiadiazole, dioxazole, dithiazole, tetrazole, oxatetrazole, thiatetrazole or pentazole.

The iNKT stimulator of formula (II) as defined above is advantageously soluble aqueous-methanol and/or DMSO solvents.

A preferred iNKT stimulator for example has the following formula (IV):

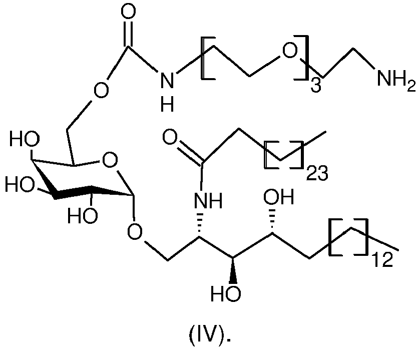

The iNKT stimulator of formula (IV) induces a high CD8+ response, resulting in a high cytotoxic response

Another preferred iNKT stimulator for example has the following formula (V):

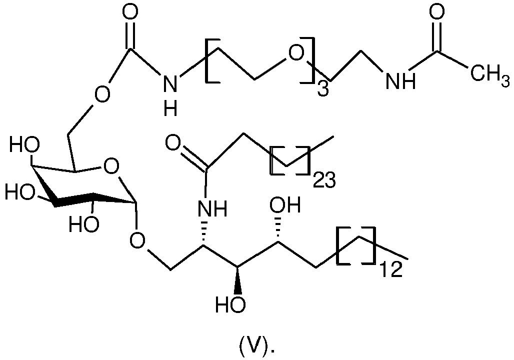

Another preferred iNKT stimulator for example has the following formula (VI):

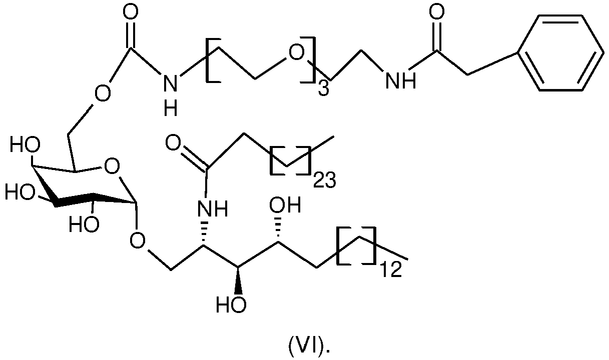

Another preferred iNKT stimulator for example has the following formula (VII):

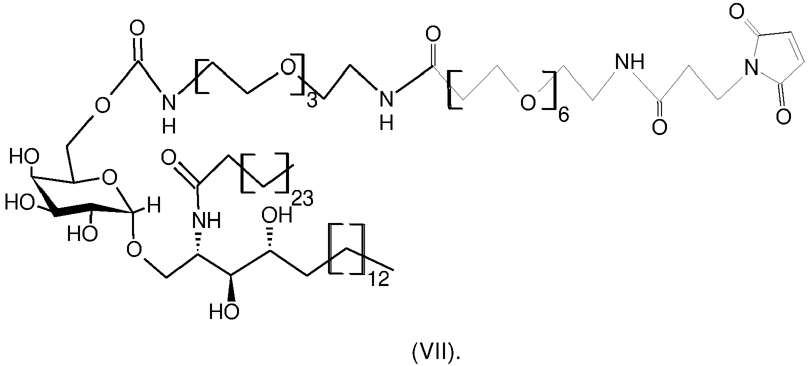

Another preferred iNKT stimulator for example has the following formula (VIII):

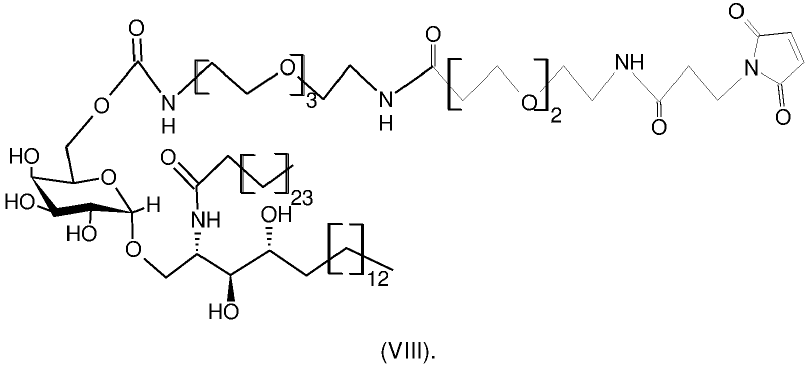

The iNKT stimulator is thus preferably selected from the group consisting of the compound of formula (IV), the compound of formula (V), the compound of formula (VI), the compound of formula (VII) and the compound of formula (VIII), as defined above.

The iNKT stimulator as defined above may comprise a carrier D, for example when R1 represents a PEG fragment -[CH₂-CH₂-O]ₚ-L-D with p being an integer from 1 to 24 or an alkyl chain -(CH₂)_{q}-L-D with q being an integer from 1 to 24, or may further be coupled to a carrier D, for example when R2 represents a functional reacting group.

The iNKT stimulator may be provided in the form of a vector comprising said iNKT stimulator, in particular as defined below.

The iNKT stimulator may be provided in the form of a pharmaceutical composition, in particular as defined below.

The iNKT stimulator is for example obtained by a process as defined below.

### Method for producing compound 6-PEGm-NHR-GalCer

The present invention also relates to a method for producing an iNKT stimulator of formula (II) as defined above, wherein said process comprises:
- optionally, reacting galactose and phytosphingosine to obtain the compound having the following formula (IX): 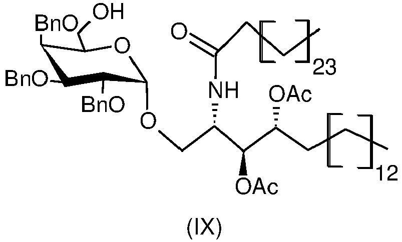 wherein said reaction preferably comprises:
   - preparing a glycosyl donor by orthogonal protections and anomeric activation of galactose,
   - preparing a glycosyle acceptor by orthogonal protection of phytosphingosine followed by *N*-acylation and deprotection steps, and
   - carrying out a glycosylation step and deprotection of primary hydroxyle,
- reacting the compound of formula (IX) with N₃-CH₂-CH₂-PEG₃-N=C=O, to obtain the compound having the following formula (X) after hydrogenation: 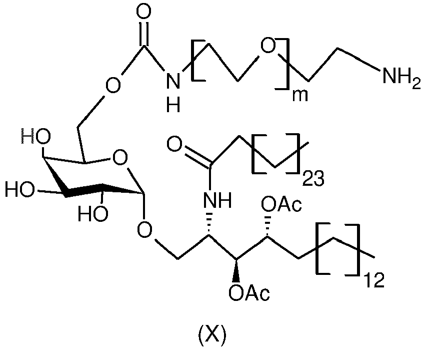
- optionally, reacting the compound of formula (X) with
   ∘ sodium methoxide, for producing the compound of formula (IV),
   ∘ acetic anhydride followed by sodium methoxide, for producing the compound of formula (V), or
   ∘ phenylacethyl chloride followed by sodium methoxide, for producing the compound of formula (VI), and
- optionally, reacting the compound of formula (IV) with a SM(PEG)₆ crosslinker, for producing the compound of formula (VII) or with a SM(PEG)₂ crosslinker, for producing the compound of formula (VIII).

Examples of method for producing an iNKT stimulator of formula (IV), (V), (VI), (VII) or (VIII) are further detailed in Example 1.

### Carrier

The present invention also relates to an iNKT stimulator as defined above comprising or coupled to at least one carrier.

The carrier is herein also referred to as "carrier D" or "D".

When the iNKT stimulator comprises or is coupled to one or at least one carrier D, it is also referred to as a complex, conjugate, bioconjugate or glycoconjugate.

The carrier may be any compound able to improve the efficiency and/or stability and/or the half-life of the iNKT stimulator, able to localize the iNKT stimulator (for example a labeling agent), able to specifically deliver the iNKT stimulator (for example a targeting agent) and/or having a therapeutic activity (for example a therapeutic agent).

The carrier is preferably a targeting agent and/or a therapeutic agent.

The targeting agent is any compound allowing delivering the iNKT stimulator as defined above to target cells and/or in an area around or close to target cells. The target agent is for example a compound binding to a receptor expressed by CD1d cells.

For example, in the frame of the prevention and/or treatment of cancer, the targeting agent may specifically bind to tumor cell receptors, in particular those expressed on CD1d cells, or to cells present in the tumor environment.

The therapeutic agent is any compound having a therapeutic effect, in particular a prophylactic and/or curative therapeutic effect. The therapeutic agent preferably has a therapeutic effect on the same disease, but preferably by a mechanism of action different from those of the iNKT.

The targeting agent and/or therapeutic agent may for example be a protein, a peptide, a glycoprotein, aptamer, a virus, a chemical molecule, preferably a small chemical molecule, or a carbohydrate.

Non-limitative examples of targeting agent are an antibody, an antibody fragment (such as a Fab, scFv, nanobody), sugar, lectin, affitin, growth factor or a chemical molecule specifically binding to a target.

Non-limitative examples of therapeutic agent are an antigen, such as a bacterial antigen or a viral antigen, an antibody, an antibody fragment or a chemical molecule (for example a synthesized bioactive molecule).

By the expression "small chemical molecule", it is herein meant a chemical molecule having a molecular weight equal to or lower than 500 kDa.

The antigen as defined above may be a carbohydrate antigen or a peptidic antigen

A given carrier may thus be both a therapeutic and targeting agent, for example such as in the case of an antibody.

The carrier may be an internalized carrier or a non-internalized carrier.

By "internalized carrier", it is herein meant a carrier which, upon binding to a target cell, is internalized in said target cell.

The internalized carrier as defined above is preferably both a therapeutic and targeting agent.

The internalized carrier as defined above is preferably an antibody.

In the case of a conjugate comprising at least one iNKT stimulator coupled to at least one internalized carrier, the internalized carrier is preferably able to internalize said conjugate.

When a conjugate comprising at least one iNKT stimulator coupled to at least one internalized carrier is internalized in a target cell, the iNKT stimulator is then preferably released from the conjugate, thereby allowing loading on CD1d molecule, presentation to the surface of target cell and iNKT stimulation.

When the carrier is an antibody, the antibody is preferably a monoclonal antibody.

The monoclonal antibody is preferably a human monoclonal antibody or a humanized monoclonal antibody.

The antibody as defined above may be a monospecific antibody or a multispecific antibody, such as a bispecific antibody.

The antibody as defined above may specifically bind to at least one receptor expressed on tumor cells.

Non-limitative examples of antibody that may be used as a carrier are anti-EGFR antibody (such as cetuximab or panitumumab), anti-HER2 antibody (such as traztuzumab).

An anti-EGFR antibody, such as cetuximab or panitumumab, is an example of internalized carrier.

The present invention thus also relates to a conjugate comprising at least one iNKT stimulator as defined above coupled to at least one carrier as defined above, preferably via a linker between the iNKT stimulator and the carrier.

The carrier of the conjugate is preferably a therapeutic agent and/or a targeting agent.

The coupling between the iNKT stimulator and the carrier may be covalent.

The present invention particularly relates to a conjugate as defined above, wherein at least one iNKT stimulator is covalently linked to at least one carrier, preferably in a non-enzymatic cleavable manner.

One, two, three or at least four iNKT stimulators may be coupled to one, two, three or at least four carriers to form one conjugate.

When the conjugate as defined above comprises at least two iNKT stimulators, the iNKT stimulators may be identical or different.

When the conjugate as defined above comprises at least two carriers, the carriers may be identical or different.

A preferred conjugate is a conjugate as defined above comprising at least one iNKTs simulator and one carrier (i.e. one molecule of carrier), the iNKT stimulator(s) being preferably linked to the carrier, optionally via a linker.

The linker present in the conjugate may for example result from the method for coupling the iNKT stimulator to the carrier.

The present invention for example relates to a conjugate comprising at least one (preferably one or two) iNKT stimulator coupled to at least one (preferably one) carrier, wherein said conjugate:
(i) comprises a compound of the formula (II) 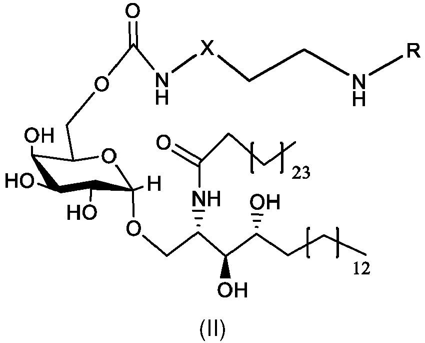 wherein X represents a PEG fragment -[CH2-CH2-O]ₘ with m being an integer from 1 to 24, an alkyl chain -(CH2)ₙ- with n being an integer from 1 to 24 or a branched hydrocarbon group, wherein R represents -CO-R1, -CONR1, -COOR1, -CSR1, -CSNR1 or -CSOR1 and wherein R1 represents a PEG fragment -[CH₂-CH₂-O]ₚ-L-D with p being an integer from 1 to 24 or an alkyl chain -(CH₂)_{q}-L-D with q being an integer from 1 to 24, wherein L is a linker and D is a carrier, or
(ii) is obtained by coupling to a carrier D an iNKT stimulator of the above formula (II), wherein X represents a PEG fragment -[CH2-CH2-O]ₘ with m being an integer from 1 to 24, an alkyl chain -(CH2)ₙ- with n being an integer from 1 to 24 or a branched hydrocarbon group, wherein R represents -CO-R1, -CONR1, - COOR1, -CSR1, -CSNR1 or -CSOR1, wherein R1 represents a PEG fragment - [CH2-CH2-O]ₚ-R2 with p being an integer from 1 to 24 or an alkyl chain -(CH2)_{q}-R2 with q being an integer from 1 to 24 and wherein R2 represents a functional reacting group allowing coupling to said carrier D.

As a non-limitative example, the coupling of a carrier which is an antibody, protein, peptide or glycoprotein may be obtained by reacting a Traut's reagent with said carrier, to obtain a Traut-thiol activated carrier, and then coupling the Traut activated carrier to the iNKT stimulator, in particular the iNKT stimulator of formula (II), wherein R is a group of formula (III) as defined above, to obtain a conjugate comprising the iNKT stimulator coupled to the carrier.

The Traut's reagent is a 2-Iminothiolane.

A still preferred conjugate is a conjugate as defined above comprising one iNKT simulator (i.e. a one molecule of iNKTs simulator) and one carrier (i.e. one molecule of carrier), the iNKT stimulator being linked to the carrier, preferably via a linker.

Another preferred conjugate is a conjugate as defined above comprising two iNKT simulators (i.e. a two molecules of iNKT simulator) and one carrier (i.e. one molecule of carrier), each of the iNKT stimulators being linked to the carrier, preferably via a linker.

The conjugate as defined above comprising at least one (preferably one, two or at least two) iNKT stimulator coupled to at least one (preferably one) carrier thus preferably comprises a compound of the formula (II) 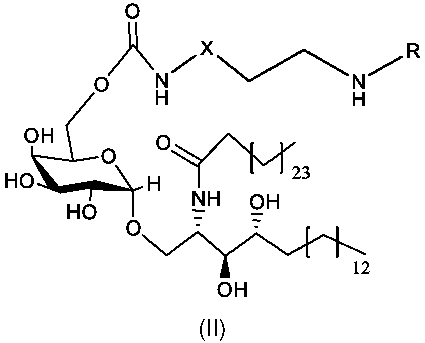 wherein X represents a PEG fragment -[CH2-CH2-O]ₘ with m being an integer from 1 to 24, an alkyl chain -(CH2)ₙ- with n being an integer from 1 to 24 or a branched hydrocarbon group, wherein R represents -CO-R1, -CONR1, -COOR1, -CSR1, -CSNR1 or -CSOR1 and wherein R1 represents a PEG fragment -[CH₂-CH₂-O]ₚ-L-D with p being an integer from 1 to 24 or an alkyl chain -(CH₂)_{q}-L-D with q being an integer from 1 to 24, wherein L is a linker and D is a carrier. The compound of formula (II) as defined above is thus of an iNKT stimulator coupled to a carrier D via a linker L. The compound of formula (II) as defined above may be coupled to at least another compound via the carrier D. Said other compound coupled to the carrier D is preferably an iNKT stimulator. Said other compound coupled to the carrier D is preferably identical to the iNKT stimulator, which is coupled to the carrier D via the linker L in the formula (II) as defined above.

The conjugate as defined above may thus have the following structure: (iNKT stimulator 1)-(Linker L1)-(carrier D)-(Linker L2)-(iNKT stimulator 2), wherein L1 and L2 are preferably identical and/or the iNKT stimulators 1 and 2 are preferably identical.

The conjugate as defined above comprising at least one (preferably one, two or at least two) iNKT stimulator coupled to at least one (preferably one) carrier may thus be obtained by coupling to one carrier D at least one (for example one, two or at least two) iNKT stimulator of the formula (II): 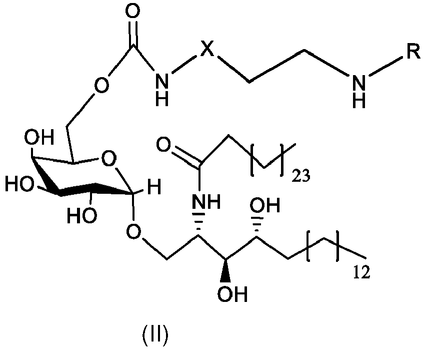 wherein X represents a PEG fragment -[CH2-CH2-O]ₘ with m being an integer from 1 to 24, an alkyl chain -(CH2)ₙ- with n being an integer from 1 to 24 or a branched hydrocarbon group, wherein R represents -CO-R1, -CONR1, -COOR1, -CSR1, -CSNR1 or -CSOR1, wherein R1 represents a PEG fragment -[CH2-CH2-O]ₚ-R2 with p being an integer from 1 to 24 or an alkyl chain -(CH2)_{q}-R2 with q being an integer from 1 to 24 and wherein R2 represents a functional reacting group allowing coupling to said carrier D.

### Vector

The iNKT stimulator as defined above or the conjugate as defined above may be provided in the form of a vector comprising said iNKT stimulator or conjugate.

The vector may be any delivery vehicle able to encapsulate a compound.

The vector may be for example a nanoparticle, such as a liposome (for example a polymeric liposome or a lipidic liposome), a Virus-Like-Particle (VLP), a dendrimer, a micelle, a nanoemulsion and nanosuspension.

The present invention thus also relates to a vector comprising at least one iNKT stimulator as defined above or at least one conjugate as defined above.

The iNKT stimulator or conjugate may be present inside the vector, in particular in the case of a liposome, dendrimer, micelle, nanoemulsion or nanosuspension.

Alternatively, the iNKT stimulator or conjugate may be on the surface of the vector, so as to be able to bind directly to CD1d expressed by the cells.

The vector may further comprise at least one targeting agent as defined above and/or at least one therapeutic agent as defined above.

### Pharmaceutical composition

The iNKT stimulator as defined above, the conjugate as defined above and/or the vector as defined above may be formulated into a pharmaceutical composition.

The present invention thus also relates to a pharmaceutical composition comprising (i) a iNKT stimulator as defined above, or a conjugate as defined above, or a vector as defined above and (ii), optionally, a pharmaceutically acceptable vehicle.

The expression "pharmaceutically acceptable vehicle" is meant to encompass any vehicle, which does not interfere with the effectiveness of the biological activity of the active ingredient and that is preferably not toxic to the host to which is administered.

Pharmaceutically acceptable vehicles can be prepared by any method known by those skilled in the art.

Suitable pharmaceutically acceptable vehicles may comprise excipients and auxiliaries, which facilitate processing of the active compounds into preparations which can be used pharmaceutically. Suitable pharmaceutically acceptable vehicles are described for example in Remington's Pharmaceutical Sciences (Mack Publishing Company, Easton, USA, 1985), which is a standard reference text in this field. Pharmaceutically acceptable vehicles can be routinely selected in accordance with the mode of administration, solubility and stability of the iNKT stimulator. For example, formulations for intravenous administration may include sterile aqueous solutions which may also contain buffers, diluents and/or other suitable additives. The use of biomaterials and/or other polymers for drug delivery, as well the different techniques and models to validate a specific mode of administration, are disclosed in literature.

The pharmaceutical composition may be a liquid, such as a solution or suspension.

The iNKT stimulator as defined above is preferably present in the pharmaceutical composition in an amount effective to achieve the intended purpose. This amount may for example depend the condition of the mammal intended for administration (e.g., weight, age, sex, health, concurrent treatment, if any, and frequency of treatment), the mode of administration and the type of formulation.

For example, the pharmaceutical composition may comprise from 5 µg to 50 mg of iNKT stimulator, preferably from 10 µg to 40 mg of iNKT stimulator, preferably 50 µg to 20 mg of iNKT stimulator, preferably from 75 µg to 10 mg, preferably from 100 µg to 5 mg, more preferably from 100 µg to 1 mg of iNKT stimulator.

The pharmaceutical composition may comprise at least one additional active ingredient.

This additional active ingredient may for example be a therapeutic agent as defined above, a drug or a prodrug.

The pharmaceutical composition may be a vaccine.

When the pharmaceutical composition as defined above is a vaccine, said composition may comprise at least one iNKT stimulator and at least one antigen, such as a virus antigen or bacteria antigen.

In one embodiment, the pharmaceutical composition is presented in a unit dosage form, in order to facilitate accurate dosing. The term "unit dosage form" refers to a physically discrete unit suitable as unitary dosage for human subjects or other mammals, each unit containing a pre-determined quantity of active material calculated to produce the desired therapeutic effect, in association with a suitable pharmaceutical vehicle. Typical unit dosage forms include pre-filled, pre-measured ampoules or syringes of the liquid pharmaceutical compositions. In such compositions, the iNKT stimulator is usually a minor component.

The invention further provides a kit comprising a pharmaceutical composition as defined above and instructions regarding the mode of administration. These instructions may e.g. indicate the medical indication, the route of administration, the dosage and/or the group of patients to be treated.

### iNKT stimulator, conjugate or vector for use as a medicament

The present invention particularly relates to an iNKT stimulator as defined above, conjugate as defined above or vector as defined above, for use as a medicament, in particular in immunotherapy, for example for the prevention and/or treatment of any disease requiring a stimulation of an immune response, in particular in a subject in need thereof.

The present invention also relates to a compound for use in a method of prevention and/or treatment of a disease requiring a stimulation of an immune response in a subject in need thereof, wherein said method comprises administering an iNKT stimulator as defined above, a conjugate as defined above or a vector as defined above to said subject.

By "disease requiring a stimulation of an immune response", it is herein meant a disease whose prevention and/or treatment requires or will be improved by an amplification or an initiation of an immune response.

The subject is for example a human being or a non-human mammal.

A human being is also referred to as an "individual" or a "patient".

Said human being may be of any age, for example an infant, child, adolescent, adult, elderly people, and of any sex.

A non-human mammal is preferably a cat, dog, rabbit, primate, mouse or rat.

The subject to be treated may suffer from or may be likely to be affected by a disease requiring a stimulation of an immune response.

The prevention of a disease requiring a stimulation of an immune response includes vaccination.

A disease requiring a stimulation of an immune response may for example be selected from the group consisting of cancer, autoimmune disease, inflammatory disease, viral infection, bacterial infection, parasitic disease and/or any immunodeficient pathology.

A cancer which may be prevented and/or treated according to the invention may be any cancer wherein the cells express CD1d, such as lung cancer, metastatic carcinoma, neck skin, head cancer, breast cancer gastric cancer, colon cancer, andenocarcinoma and/or cold tumor.

An autoimmune disease which may be prevented and/or treated according to the invention may for example be celiac disease, diabetes mellitus type 1, Graves' disease, inflammatory bowel disease, multiple sclerosis, psoriasis, rheumatoid arthritis or systemic lupus erythematosus.

An inflammatory disease which may be prevented and/or treated according to the invention may for example be chronic peptic ulcer, tuberculosis, rheumatoid arthritis, periodontitis, ulcerative colitis or Crohn's disease.

A viral infection may for example be may be any infection caused by a virus, such as Hepatitis C infection or Hepatitis B infection.

A bacterial infection may for example be may be any infection caused by bacteria.

A parasitic disease may for example be may be any infection caused by a parasite.

The iNKT stimulator is as defined above.

In particular, the iNKT stimulator may be obtained by a method as defined above.

The iNKT stimulator, conjugate or vector may be provided in the form of a pharmaceutical composition as defined above.

The iNKT stimulator may comprise or carrier as defined above or be coupled to a carrier as defined above.

Using a targeting agent as a carrier advantageously allows delivering the iNKT stimulator to the target cells expressing both the ligand of the targeting agent and CD1d, thereby allowing reducing possible side effects and/or the dose needed to reach the therapeutic effect.

For example, for the prevention and/or treatment of cancer, the iNKT stimulator is advantageously coupled to an agent targeting a specific receptor present in the tumoral environment, such as a specific receptor of the tumoral cells themselves, preferably a specific receptor of the tumoral cells expressing CD1d, or a specific receptor of cells specifically found around the tumor.

The carrier is preferably an antibody or antibody fragment, as defined above.

For example, a conjugate comprising at least one iNKT stimulator as defined above coupled to an EGFR-antibody may be used or administered for the prevention and/or treatment of cancer, preferably of colorectal cancer and/or breath cancer.

For example, a conjugate comprising at least one iNKT stimulator as defined above coupled to an EGFR-antibody may be used or administered for the prevention and/or treatment of cancer, preferably of colorectal cancer, breath cancer and/or adenocarcinoma.

For example, a conjugate comprising at least one iNKT stimulator as defined above coupled to an HER2-antibody may be used or administered for the prevention and/or treatment of cancer, preferably of breast cancer, gastric cancer, colon cancer and/or adenocarcinoma.

The conjugate as defined above comprising an anti-EGFR antibody may advantageously be used or administered for the prevention and/or treatment of cancer in a subject in need thereof, independently of if this subject carries at least one Kras and/or N-Ras mutation(s). On the contrary, cetuximab alone or Panitumumab alone are not efficient for treating patients carrying Kras and/or N-Ras mutation(s) and are only used for treating subjects carrying wild-type Kras and N-Ras genes. In the frame of the present invention, the anti-EGFR is indeed mainly used as a targeting agent.

The conjugate as defined above comprising an anti-EGFR antibody can thus advantageously be used or administered for the prevention and/or treatment of cancer in any subject in need thereof, in particular in a subject resistant to a treatment using an anti-EGFR antibody alone. Besides, the conjugate as defined above comprising an anti-EGFR antibody may thus advantageously be used or administered, without previously requiring to look for the presence of Kras or N-Ras mutation(s).

A conjugate comprising at least one iNKT stimulator as defined above coupled to an affitin or a chemical molecule, preferably a small chemical molecule, said affitin or chemical molecule being able to recognize a bacterial or viral compound (such as a bacterial or viral antigen) may be used or administered for the prevention and/or treatment of a bacterial or viral infection.

The iNKT stimulator is preferably used or administered in a therapeutically effective amount.

By "therapeutically effective amount", it is herein meant an amount sufficient to achieve an initiation or an amplification of an immune response. Such effective amount can be routinely determined by the skilled person. The amount of iNKT stimulator actually administered will typically be determined by a physician, in the light of the relevant circumstances, including the condition to be prevented or treated, the chosen route of administration, the actual agent administered, the age, sex, weight and response of the individual subject, the severity of the subject's symptoms and the like. It will also be appreciated by the skilled person that the dosage may be dependent on the stability of the administered agent.

The effective amount may also vary according to the therapeutic agent(s), drug(s) and/or prodrug(s) with which the iNKT stimulator as defined above may be co-administered.

A therapeutically effective amount encompasses an amount in which any toxic or detrimental effects of the iNKT stimulator are outweighed by the therapeutically beneficial effects.

The iNKT stimulator, conjugate, vector or pharmaceutical composition may be used or administered by any suitable route, such as the arterial route, venous route, intra-tissue route, intraperitoneal route, intranasal route, intracerebral route, ocular route and/or oral route.

The administration of the iNKT stimulator, conjugate, vector or the pharmaceutical composition comprising thereof may be achieved in a single dose or several doses, said several doses being injected simultaneously, separately or sequentially.

The iNKT stimulator, conjugate, vector or pharmaceutical composition may for example be used or administered in a therapeutic dose of from 10 µg to 4800 µg of iNKT stimulator / m² of body surface, for example from 20 µg to 2000 µg of iNKT stimulator / m² of body surface, such as from 20 µg to 500 µg of iNKT stimulator / m² of body surface or from 50 µg to 500 µg of iNKT stimulator / m² of body surface.

### In vitro use of a iNKT stimulator

The present invention also relates to the *in vitro* use of an iNKT stimulator as defined above, a conjugate as defined above or a vector as defined above to activate iNKT cells, in particular for inducing or increasing the expression of at least one cytokine as defined above.

The present invention particular relates the *in vitro* use of an iNKT stimulator as defined above, a conjugate as defined above or a vector as defined above for inducing a stimulation and/or amplification of immune CD1d-dependent iNKT response.

The iNKT stimulator as defined above, conjugate as defined above or vector as defined above may thus be used in any assay wherein an *in vitro* stimulation and/or amplification of immune CD1d-dependent iNKT response is needed.

The invention will be further illustrated by the following figures and examples. However, these examples and figures should not be interpreted in any way as limiting the scope of the present invention.

### Figures

Figure 1: Expression of CD1d on HeLa cells in comparison with their respective CD1d transfectants.
Figure 2: IFN-γ, IL-2 and IL-13 secretions by IKNT cells after activation with HEK293 (A) or HeLa (B) cells and their respective CD1d transfection models, loaded with KRN7000.
Figure 3: IFN-γ, IL-2 and IL-13 secretions by IKNT cells after activation with HEK293 (A) or HeLa (B) cells and their respective CD1d transfection models, loaded with 6-PEG₃-NH₂-GalCer (2a).
Figure 4: IFN-γ secretion after activation of iNKT cells by either PBMCs or non-CD1d-HEK293 cells loaded with 6-PEG₃-NH₂-GalCer (2a).
Figure 5: CD1d-dependancy of the iNKT cells activation observed using both HEK293 CD1d-transfected (left panel A) and control HEK293 cells (right panel B) as antigen presenting cells. IL-2 secretion induced by 6-PEG₃-NH₂-GalCer (2a) in the presence or absence of anti-CD1d antibody.
Figure 6: RT-PCR analysis of CD1d expression on cells lines used as antigen presenting cells (SW620, non-transfected HEK293 and HEK293 transfected to express CD1d).
Figure 7: Cytotoxocity induced by iNKTs on HeLa-CD1d cells (expressed as percentage of dead cells) after activation with 6-PEG₃-NH₂-GalCer (2a) or KRN7000.
Figure 8: iNKT phenotyping. Expansion of iNKT cells in PBMC in the presence of KRN7000 or 6-PEG₃-NH₂-GalCer (2a) expressed as the ratio CD8+/CD4+.
Figure 9: Cytotoxocity induced by iNKTs on HeLa-CD1d spheroid models, after activation with 6-PEG₃-NH₂-GalCer (2a) or KRN7000. Experiment protocol (A) and cells death analysis by flow cytometry (B).
Figures 10 and 11: IFN-γ and IL-13 secretion by iNKTs after activation with 6-PEG₃-NHAc-GalCer (2b), 6-PEG₃-NH₂-GalCer (2a), 6-PEG₃-NHBz-GalCer (2c) and KRN7000.

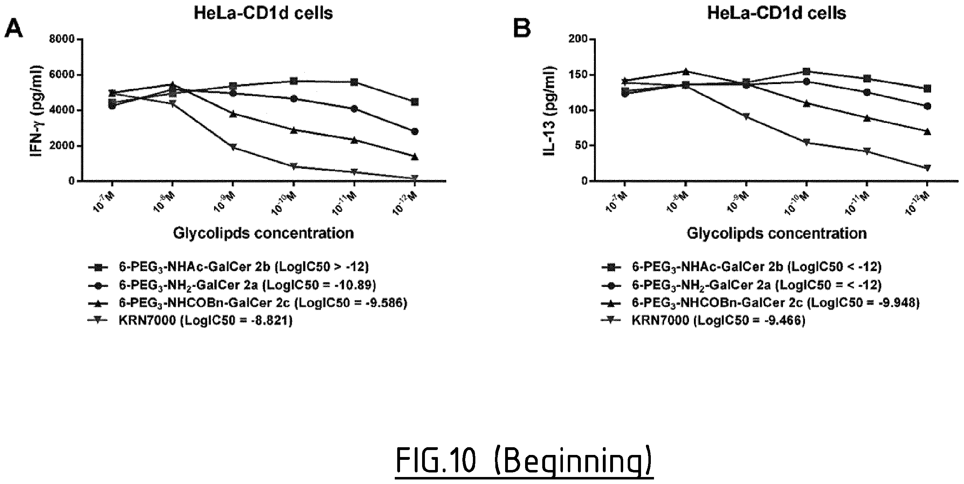

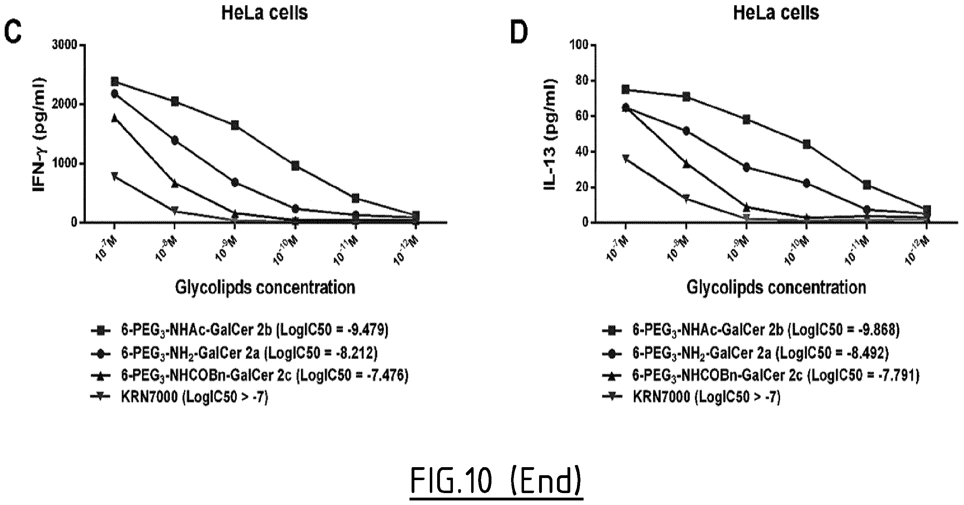

Figure 10: IFN-γ secretion with HeLa-CD1d cells (A) and non CD1d HeLa cells (C) and IL-13 secretion by iNKTs after activation with HeLa-CD1d cells (B) and non CD1d HeLa cells (D).

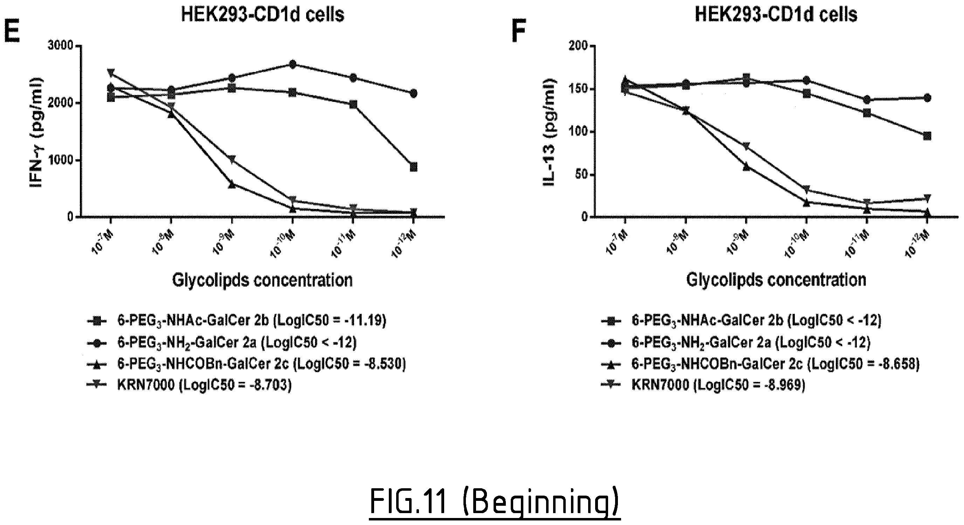

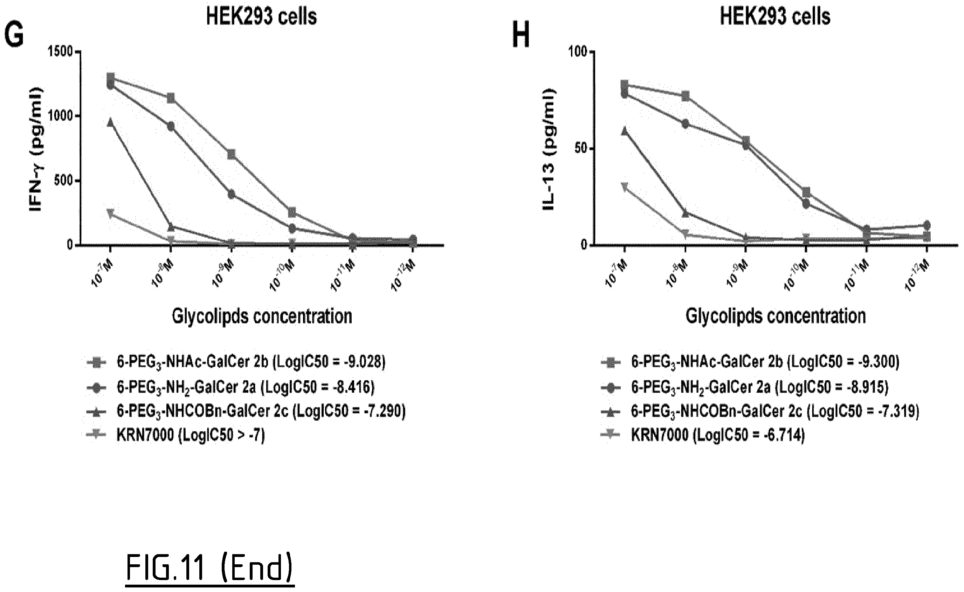

Figure 11: IFN-γ secretion with HEK293-CD1d cells (E) and non CD1d HEK293 cells (G) and IL-13 secretion by iNKTs after activation with HEK293-CD1d cells (F) and non CD1d HEK293 cells (H).

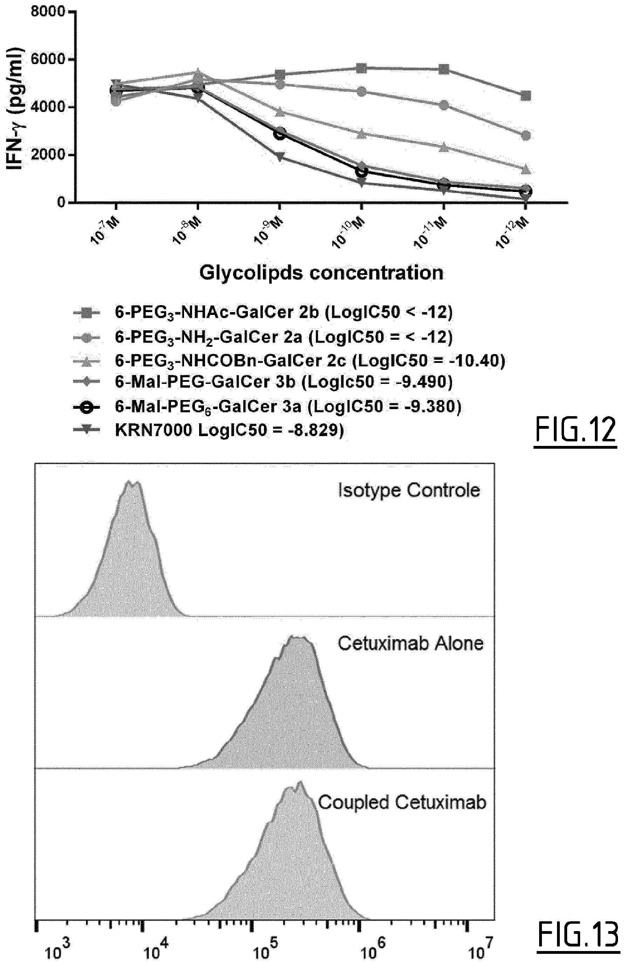

Figure 12: IFN-γ secretion by iNKTs after activation with HeLa cells loaded with 6-PEG₃-NHAc-GalCer (2b), 6-PEG₃-NH₂-GalCer (2a), 6-PEG₃-NHBz-GalCer (2c), 6-Mal-PEG-GaICer (3b), 6-Mal-PEG₆-GalCer (3a)and KRN7000.
Figure 13: EGFR recognition by cetuximab purified after the coupling ("Coupled Cetuximab") or the "mock" coupling ("Cetuximab alone") reactions.

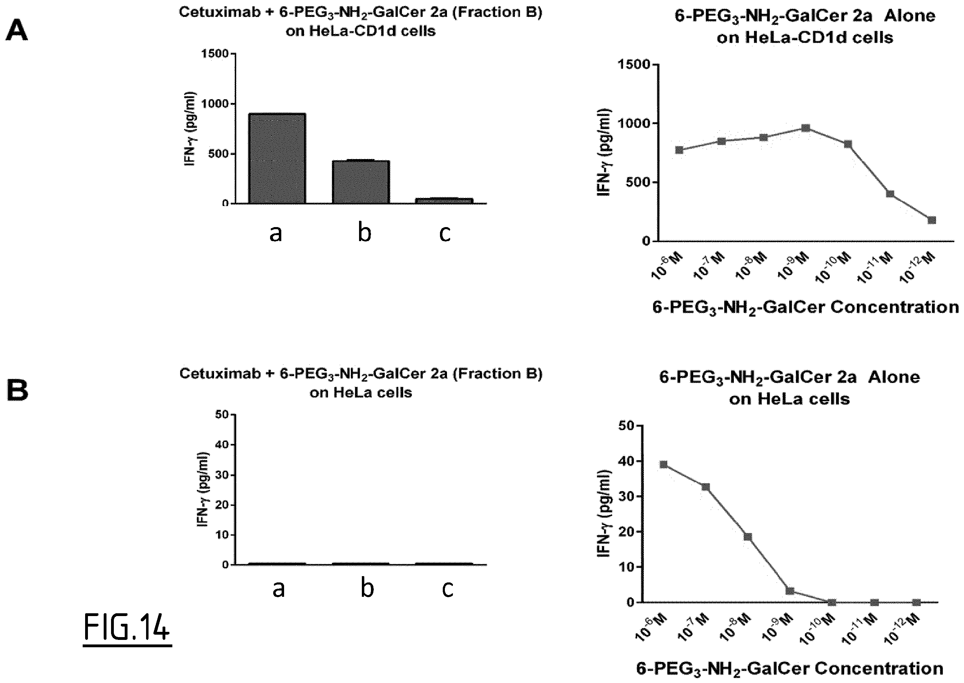

Figure 14: IFN-γ secretion by iNKTs after activation with HeLa-CD1d cells (A) or non transfected HeLa cells (B) loaded with Cetuximab + 6-PEG₃-NH₂-GalCer (2a) (left panel) or with 6-PEG₃-NH₂-GalCer (2a) alone (right panel).

**Table 1**

| Figure 14, A, left panel | a | b | c |
|---|---|---|---|
| Concentration of coupling product | 10 µg/ml | 1 µg/ml | 0,1 µg/ml |
| Starting concentration | 10⁻⁷M | 10⁻⁸M | 10⁻⁹M |
| Observed concentration | 10⁻¹⁰M | 10⁻¹¹M | 10⁻¹²M |

**Table 2**

| Figure 14, B, left panel | a | b | c |
|---|---|---|---|
| Concentration of coupling product | 10 µg/ml | 1 µg/ml | 0,1 µg/ml |
| Starting concentration | 10⁻⁷M | 10⁻⁸M | 10⁻⁹M |
| Observed concentration | <10⁻¹⁰M | <10⁻¹⁰M | <10⁻¹⁰M |

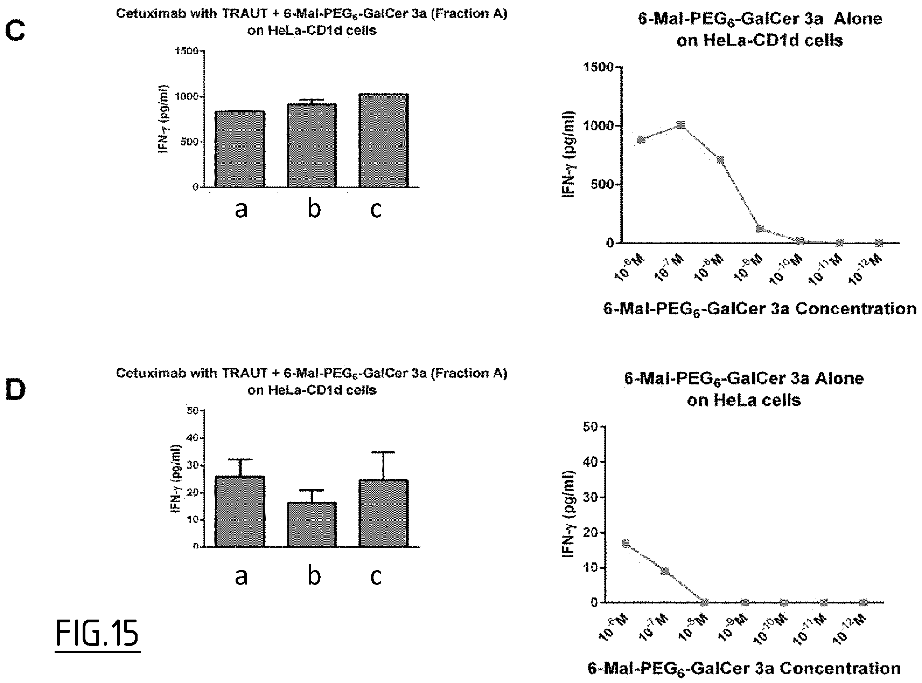

Figure 15: IFN-γ secretion by iNKTs after activation with HeLa-CD1d cells (C) or non transfected HeLa cells (D) loaded with the complex Cetuximab 6-Mal-PEG₆-GalCer (3a) formed by TRAUT activation (left panel) or with 6-Mal-PEG₆-GalCer (3a) alone (right panel).

**Table 3**

| Figure 15, C, left panel | a | b | c |
|---|---|---|---|
| Concentration of coupling product | 10 µg/ml | 1 µg/ml | 0,1 µg/ml |
| Starting concentration | 10⁻⁷M | 10⁻⁸M | 10⁻⁹M |
| Observed concentration | >10⁻⁸M | >10⁻⁸M | >10⁻⁸M |

**Table 4**

| Figure 15, D, left panel | a | b | c |
|---|---|---|---|
| Concentration of coupling product | 10 µg/ml | 1 µg/ml | 0,1 µg/ml |
| Starting concentration | 10⁻⁷M | 10⁻⁸M | 10⁻⁹M |
| Observed concentration | >10⁻⁶M | >10⁻⁶M | >10⁻⁶M |

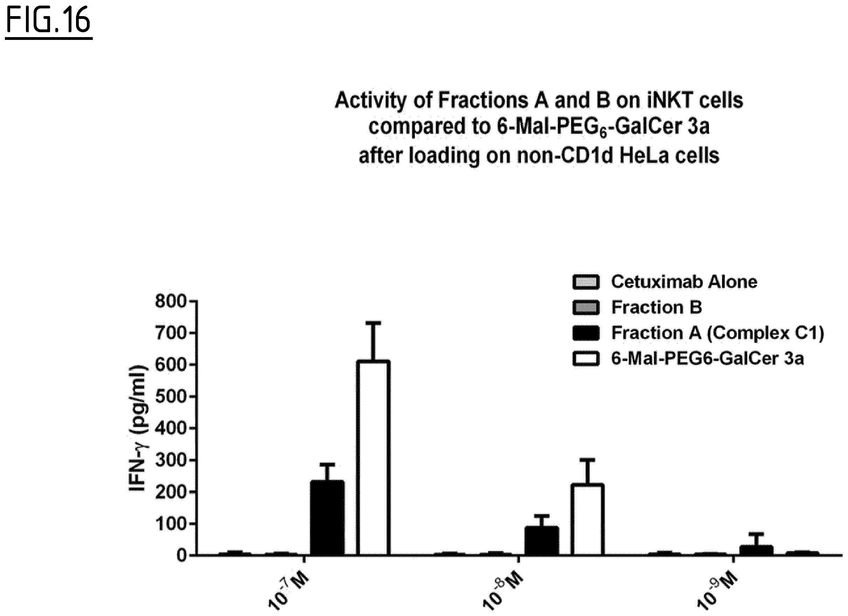

Figure 16: Activity of fractions A and B on iNKT cells compared to 6-Mal-PEG₆-GalCer 3a after loading on non-CD1d HeLa cells.

### EXAMPLES

### Example 1: Synthesis of 6-PEG-NHR-GalCer 2 and 6-Mal-PEGₙ-GalCer 3

The synthesis of compounds 6-PEG₃-NHR-GalCer of formula IV (2a), V (2b), VI (2c) (R = H, Ac and PhCH₂CO, respectively) and 6-Mal-PEGₙ-GalCer VII (3a) and VIII (3b) is based on conventional chemical pathway described in the literature.

The synthesis of key protected 6"OH-Galcer intermediate to produce 6-PEG₃-NHR-GalCer 2 and 3 was achieved from galactose and phytosphingosine precursors, by slightly modified established chemical procedures (*see Scheme 1 below*). 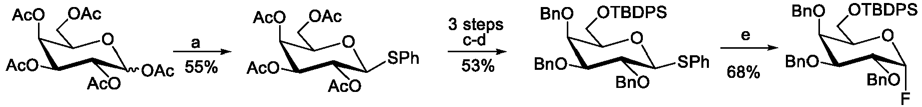 a) PhSH, BF₃.Et₂O, DCM, MS4Å ; c) MeONa/MeOH ; d) TBDPSCI, Et₃N, Pyr. ; e) NaH, BnBr, DMF/THF ; f) NBS, DAST, DCM, -15°C. 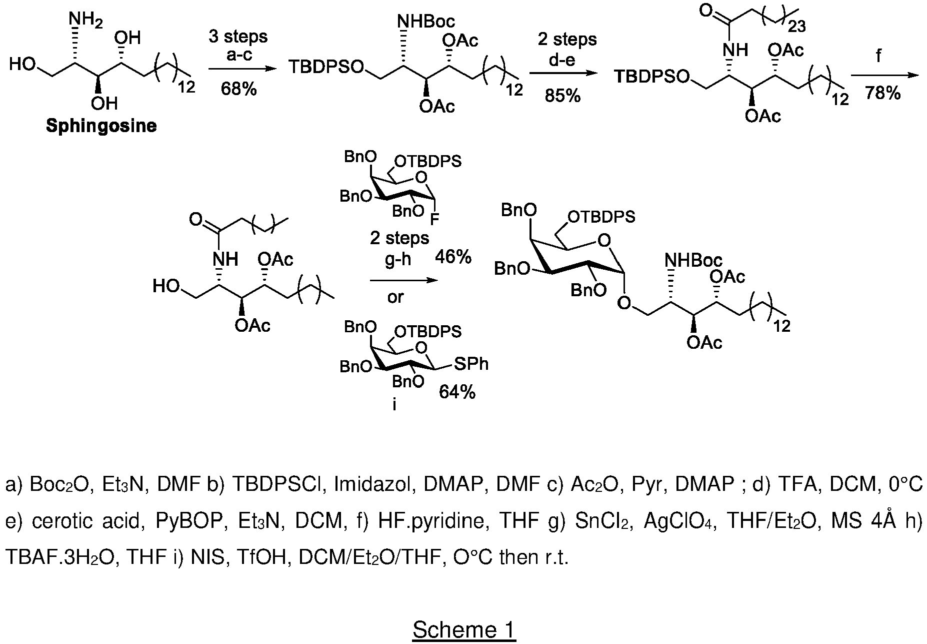 From the protected 6"-OH-Galcer, all 6-PEG₃-NHR-GalCer 2 and 6-Mal-PEGₙ-GalCer 3 are accessible (see Schemes 2 and 3 below). 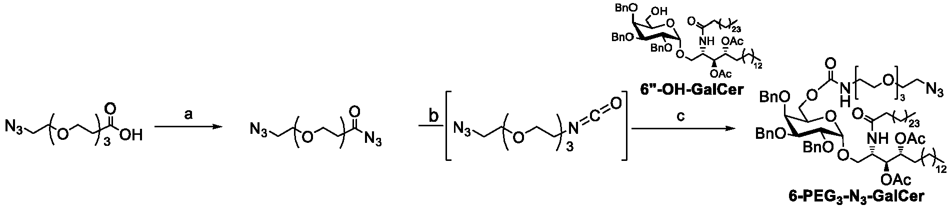 a) ClCO₂Et, Et₃N, THF, 0°C to rt, 30 min, then NaN₃, H₂O/THF, 0°C to rt, 1 h quant. b) Toluene, 90°C, 15 min c) DABCO, toluene, reflux, 4h, 86%

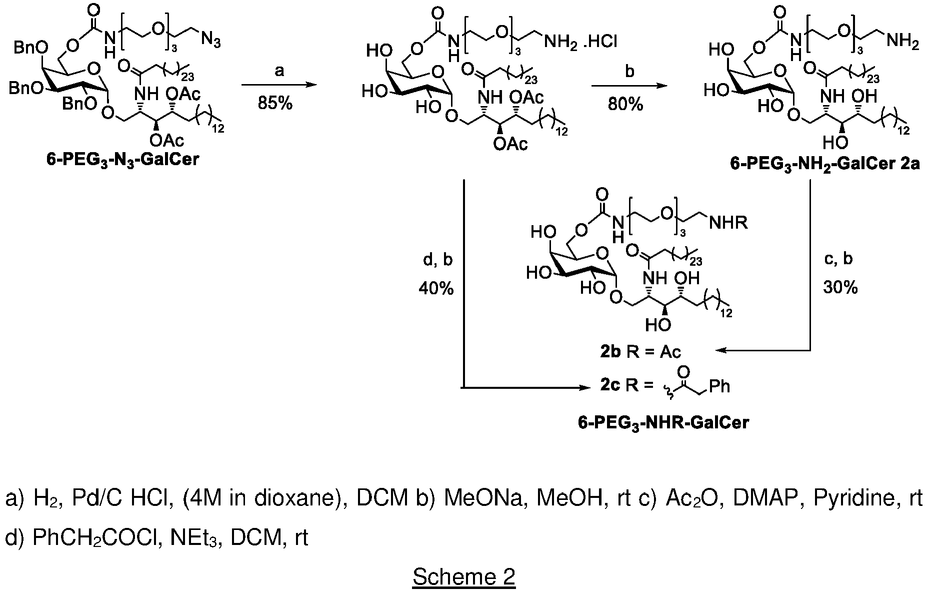

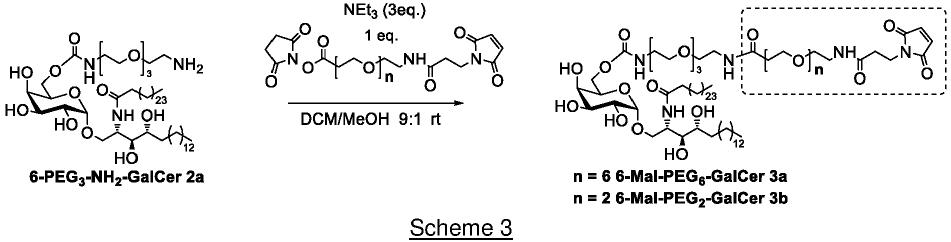

### Example 2: Activation of iNKTs by 6-PEG₃-NHR-GalCer 2 and 6-Mal-PEGₙ-GalCer 3

To analyse ability of 6-PEG₃-NHR-GalCer 2 and 6-Mal-PEGₙ-GalCer 3 to activate iNKTs, several types of presenting cells were used, such as HEK293 or HeLa cells transfected to express CD1d molecule on their membrane. Non-transfected cells were also used as negative control "at first glance" since presentation of glycolipids to iNKT cells is known to be dependent on CD1d.

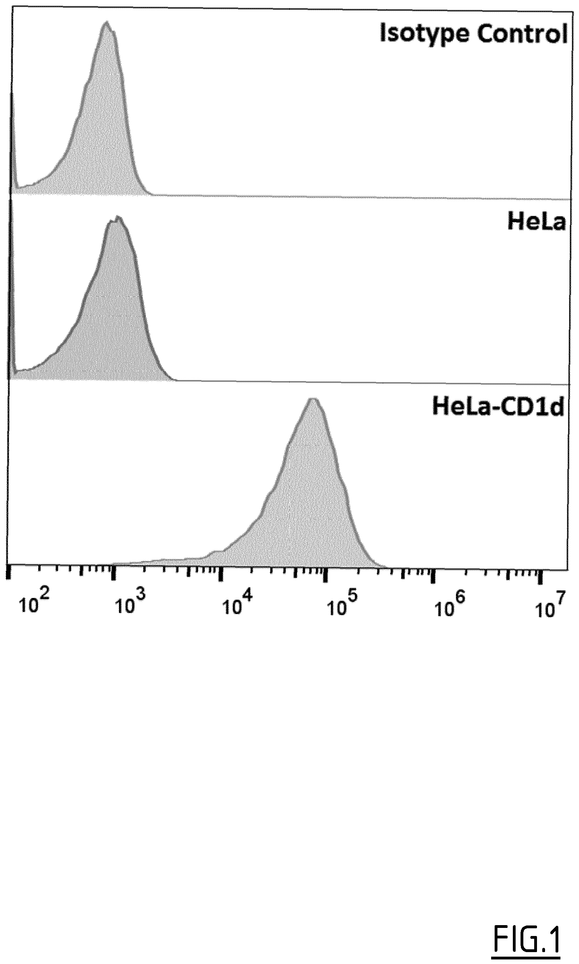

CD1d expression on HEK293 and HeLa +/- CD1d was analyzed by flow cytometry (Figure 1). Cells were labeled with an anti-CD1d-FITC or with the associated isotype control for 20min and washed to be read on Accuri C6 Flow Cytometer. A shown in Figure 1, in both cases non transfected cells appeared negative, notably when compared with their CD1d-transfected counterparts.

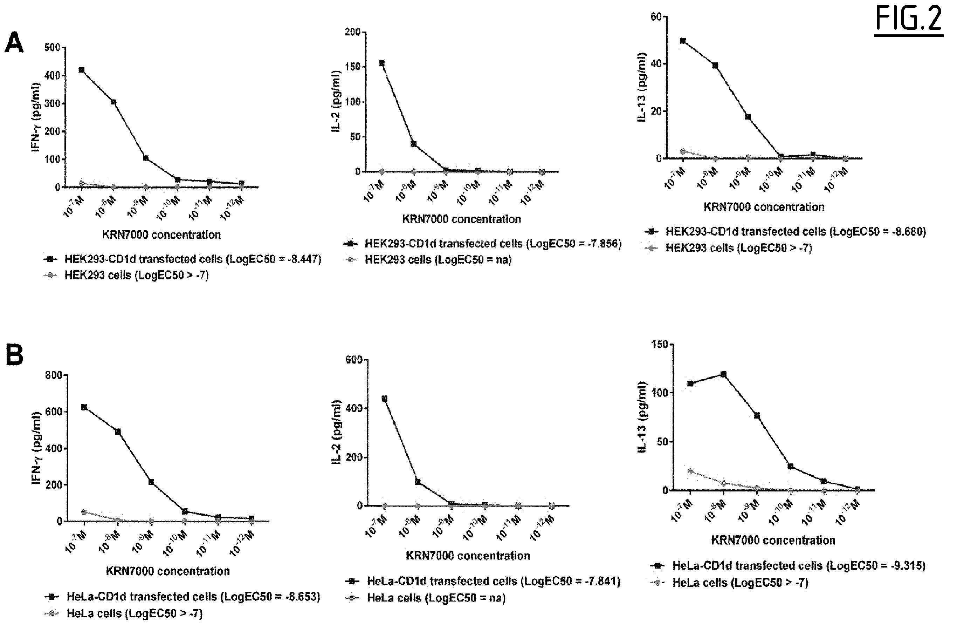

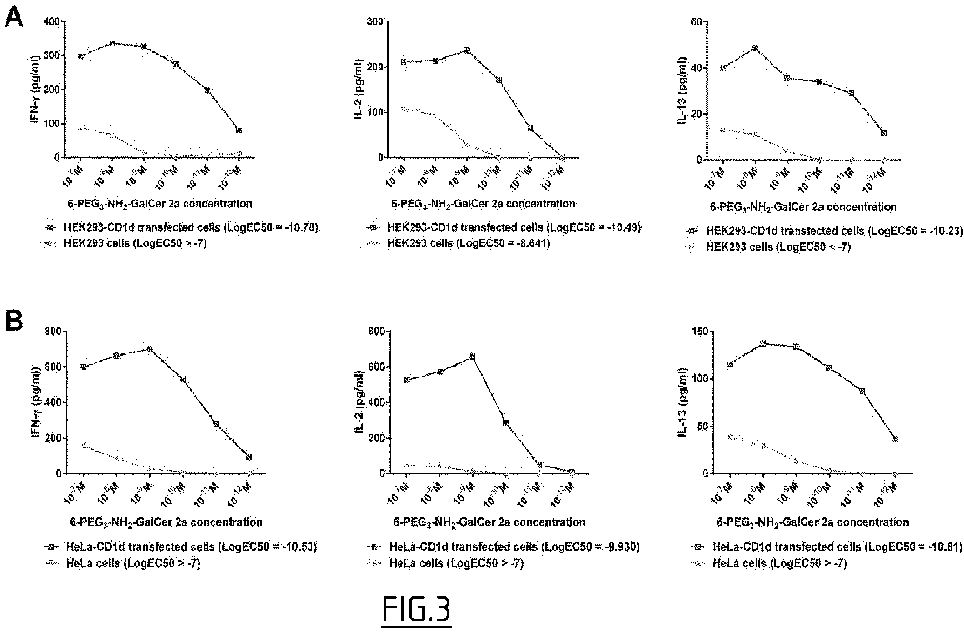

These cells were then used as antigen presenting cells (APC) to compare the iNKT cell activation potency of the canonical α-GalCer (KRN7000) ligand to that of the analogue 6"-modified with an aminoPEG linking arm 6-PEG₃-NH₂-GalCer 2a. APC were loaded with glycolipids after co-incubation at various concentrations overnight. Next day, cells were washed and co-cultured with iNKTs (2 APC for 1 iNKT). After 6 hours, supernatants were collected, and cytokines secretions (IFN-y and IL-2 for Th1 panel and IL-13 for Th2 panel) was measured by ELISA (Figure 2 and 3)

Firstly, canonical ligand, KRN7000, was presented to iNKT cells by HEK293 (Figure 2A) or HeLa (Figure 2B) cells and the associated CD1d transfected model. In both cases, KRN7000 induced IFN-γ, IL2 and IL13 releases (LogIC50 are recapitulated in table 1), in the presence of a high expression of CD1d on CD1d-transfected cells, underscoring the CD1d-dependency of the glycolipid recognition by iNKT cells. When 6-PEG₃-NH₂-GalCer 2a was used (Figure 3A and 3B), we observed a much stronger IFN-γ, IL13 and IL-2 secretions at almost 100 fold higher than KRN7000 (See comparisons in table 1).

But more surprisingly, in absence of CD1d (tests carried out on non-CD1d Hela and HEK293 cells), still a significant dose response secretion of cytokines was detected while KRN7000 remains very poorly active or ineffective. These unexpected results were not observed when using SW620 cells as control (absence of CD1d, data not shown).

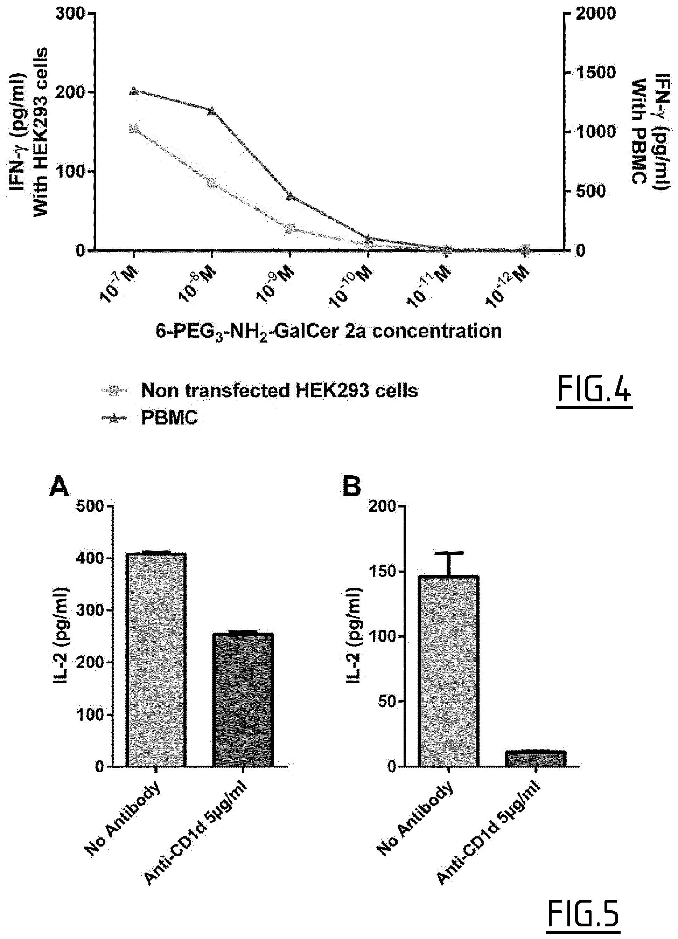

A similar cytokine release profile was confirmed with IFN-γ released from PBMC cells, which represent a closer human medium, compared from non-CD1d- HEK293 cells (see Figure 4).

This result indicates that 6-PEG₃-NH₂-GalCer 2a appears among the most powerful activators of h-iNKT known to date. These biological outcomes of the 6-PEG₃-NH₂-GalCer 2a makes this novel candidate very interesting in the context of immunotherapy against cancer regarding the development of the clinical trial with KRN7000.

**Table 5**

| **HEK293-CD1d** | **KRN7000 LogEC50** | **6-PEG₃-NH₂-GalCer LogEC50** | **Delta LogEC50** |
|---|---|---|---|
| **IFN-γ** | **-8.447** | **-10.78** | **2.333** |
| **IL-2** | **-8.680** | **-10.23** | **1.55** |
| **IL-13** | **-7.956** | **-10.49** | **2.534** |
| | | | |

| **HeLa-CD1d** | **KRN7000 LogEC50** | **6-PEG₃-NH₂-GalCer LogEC50** | **Delta LogEC50** |
|---|---|---|---|
| **IFN-γ** | -8.653 | -10.53 | 1.877 |
| **IL-2** | -9.315 | -10.81 | 1.495 |
| **IL-13** | -7.841 | -9.930 | 2.089 |

### Example 3: "non-CD1d" tumor cells: a wrong paradigm

In light of the intriguing observations of an INKT activation on "non-CD1d" tumor cells, it was investigated how 6-PEG₃-NH₂-GalCer 2a could activate iNKT cells when seemingly CD1d negative cells. Non-transfected HeLa and HEK293 cells were thus used as auto-antigens presenting cells. The hypothesis made was about the existence of a pool of CD1d, even very low, at the surface of the non-CD1d tumor cells but sufficient to be recognized by the high potent 6-PEG₃-NH₂-GalCer a2 glycolipid allowing an activation of iNKT, while KRN7000 fails to achieve this goal.

It was then checked whether iNKT cells activation might be provided by a very low expression of CD1d on the proper non-CD1d tumor cells that cannot be detected by flow cytometry. To this aim, the same activation experiments were performed but in presence of an anti-CD1d antibody. HEK293 cells and HEK293-CD1d were loaded with 6-PEG₃-NH₂-GalCer a2 as described previously. Before co-culture with iNKTs cells, loaded presenting cells were co-incubated with the anti-CD1d antibody for 1 hours, iNKTs were added directly in suspension and co-cultured 6 hours before cytokine analysis in supernatant (ELISA). If activation signal was provided by presentation of the modified glycolipid through CD1d, it was expected to observe a blockage, or at least a decrease, of cytokine secretion after blocking the signal with the anti-CD1d antibody.

When CD1d-HEK293 positive cells were used, only an uncomplete inhibition was observed in the presence of the antibody (see Figure 5A). This is likely due to the very high potency of 6-PEG₃-NH₂-GalCer 2a combined with the high level of CD1d expression on the transfected cells. However, the cytokine signal was almost completely eliminated when using the "non CD1d" cells (see Figure 5B). This experiment clearly suggests the existence of an, until now, unknown low level of CD1d molecules on non-transfected HEK293 cells.

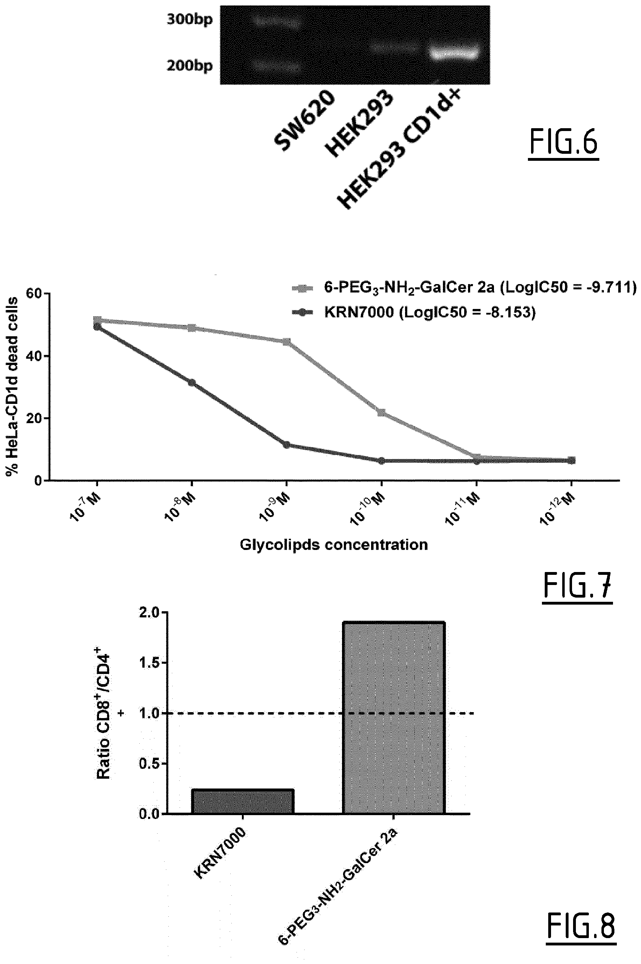

To confirm this hypothesis, the most sensitive method to detect low signal of CD1d is to determine the ARN expression of CD1d by using PCR. Three cells lines were analysed, HEK293 with CD1d as positive control, SW620 that do not activate iNKTs cells with 6-PEG₃-NH₂-GalCer 2a as negative control and non-transfected HEK293 cells (see Figure 6).

As shown in Figure 6, HEK293-CD1d positive cells showed a strong CD1d signal whereas the SW620 cells proved to be CD1d-negative, consistent with their inability to function as antigen presenting cells of the 6-PEG₃-NH₂-GalCer compound 2a. Interestingly, for non-CD1d-HEK293 cells a significant signal was detected. This confirmed, contrary to the paradigm, that these cells express a low level of CD1d, not sufficient to induce iNKTs activation using KRN7000, but sufficient to be activate with 6-PEG₃-NH₂-GalCer 2a, 100 to 1000 fold more powerful.

These results clearly indicate that 6-PEG₃-NH₂-GalCer 2a unexpectedly turns out to be far more potent than KRN7000 although similarly dependent upon CD1d presentation.

Another interesting point is the ability of 6-PEG₃-NH₂-GalCer 2a to remain efficient even at a very low level of CD1d expression in tumor cells. This redefines the notion of non-CD1d cells in anticancer treatments, since these results showed that tumor cells previously considered as CD1d negative constitutively express a minor level of CD1d, sufficient to induce activation of iNKT cells when loaded with the very potent 6-PEG₃-NHR-GalCer glycolipids.

This suggests that in order to initiate an immune response in the tumor environment, cancer cells might be used as presenting cells to activate iNKT cells in the presence of these potent glycolipids, bypassing the requirement for classical CD1d-antigen presenting cells (such as monocytes, macrophages or dendritic cells and lymphocyte B). To confirm this discovery, various other colon, lung, embryonic kidney, cervical cancer cells lines were screened by RT-PCR to define those that express small levels of CD1d and to confirm their ability to self-present the new potent iNKT agonist (see Table 6).

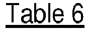

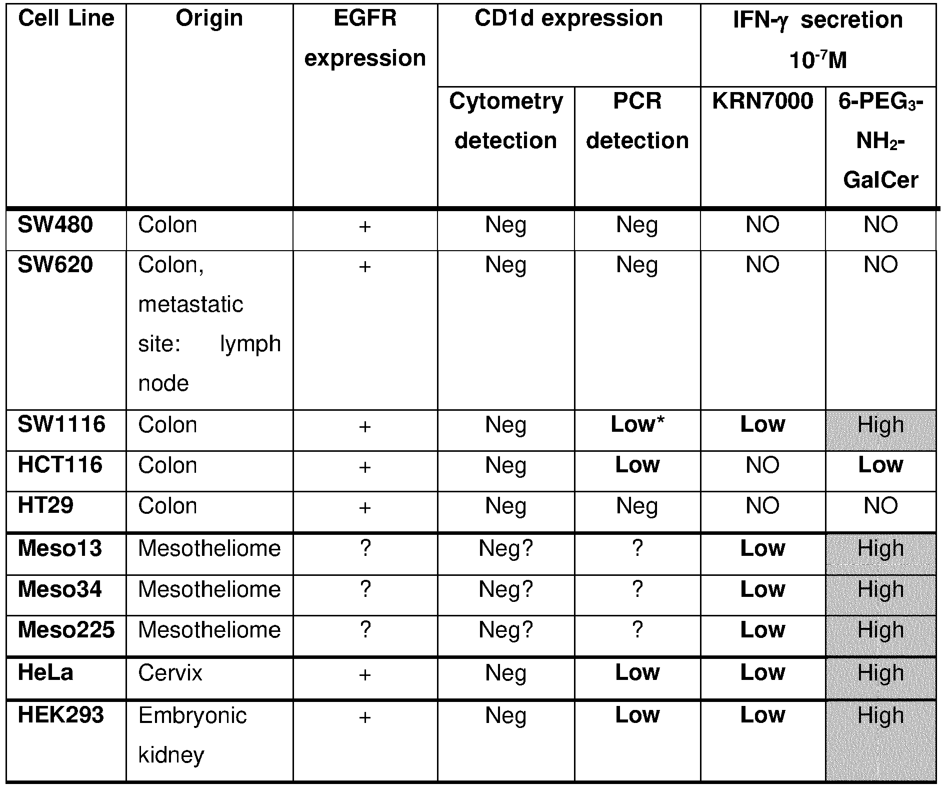

Moreover, 6-PEG₃-NH₂-GalCer 2a is not only one of a strongest activator of iNKTs cells known for cytokine secretions, but that he is also able to induce a better iNKT cytotoxicity effect than KRN7000. iNKT were co-cultured with HeLa CD1d transfected cells for 24 hours in presence of various concentration of glyclipids, then, mortality of target HeLa-CD1d cells was analysed by Flow Cytometry. As shown in Figure 7, 6-PEG₃-NH₂-GalCer 2a appear at least, 10 fold more efficient than KRN7000 to induce cytotoxicity.

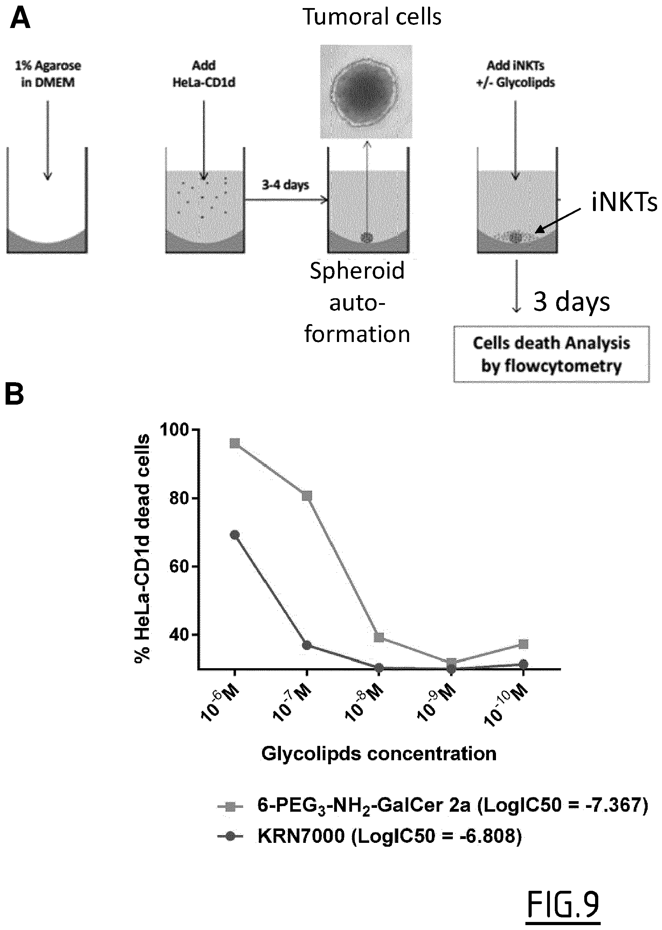

Comparative study was run to establish either a CD4 or CD8 orientation of the immune response can be induced using 6-PEG₃-NH₂-GalCer 2a vs KRN7000 (see Figure 8). PBMC were cultured in presence of an high dose of KRN or 6-PEG₃-NH₂-GalCer 2a, after ten days, phenotype of iNKTs was analysed by Flow Cytometry. Very interestingly it seems that 6-PEG₃-NH₂-GalCer 2a induces a higher cytotoxic CD8+ response from PBMC cells than KRN7000 which seems to not distinguish CD4+ to CD8+ cells stimulation. This result already established the immunocytotoxicity efficiency of 6-PEG₃-NH₂-GalCer 2a via iNKT stimulation. This ability was confirmed by an other cytotoxic assay (for 72 hours) on HeLa-CD1d 3D spheroid model (Figure 9 A) an other more complex and more resistant model in which, one more time, 6-PEG₃-NH₂-GalCer 2a appear more efficient than KRN7000 to induce cell death (preliminary data, see Figure 9B).

### Example 4: Evaluation of 6-PEG₃-NHR-GalCer 2b and 2c analogues of 6-PEG₃-NH₂-GalCer 2a

In order to better understand the surprising iNKT stimulation potency of 6-PEG₃-NH₂-GalCer 2a and the influence of end terminal amino function of the 6"-O-PEG substituted KRN7000 analogues, it has been envisioned to block the NH₂ group by an acetate 2b and a benzocarbonyl 2c groups. It was expected to establish if a suspected stabilisation of the ternary CD1d-Tumor cells/6-PEG₃-NH₂-GalCer/TCR-iNKT complex would be explained by the presence of a free amine at the end of the spacer 14 atoms chain or if the PEG sequence allows some variations at the terminal group without a loss of performance.

Two new protected NHR analogues of 6-PEG₃-NH₂-GalCer 2a were thus prepared, wherein R is an acetate 6-PEG₃-NHAc-GalCer 2b or a benzoyl group 6-PEG₃-NHCOBn-GalCer 2c. In same end, two activated derivatives 6-Mal-PEGₙ-GalCer 3a and 3b (n = 6 or 1, respectively) were also prepared with the aim to be engaged in a linkage with a biological carrier.

Both end terminal NHR protecting groups were though to avoid or alter the interaction that could occurred with CD1d-tumor cells and the TCR receptor in the presence of a free NH₂ terminal group.

6-PEG₃-NHR-GalCer analogues of 6-PEG₃-NH₂-GalCer 2a were evaluated on transfected CD1d and non-CD1d Hela and HEK293 cells for secretion of INF-y and IL13 stimulation (see Figures 10 and 11).

Depending on the model, 6-PEG₃-NHAc-GalCer 2b appears almost 10 to 100 fold more potent to activate iNKTs on CD1d transfect tumor cells (LogIC₅₀> -12 for IFN-γ on HeLa CD1d transfected cells), than PEG₃-NH₂-GalCer 2a (LogIC50=-10.89) its self and almost 10⁴ higher than KRN7000 (LogIC50 = -8.8) whereas 6-PEG₃-NHCOBn-GalCer 2c have a closer cytokine secretion potency than KRN7000.

These data are confirmed on non-CD1d-tumor cells that PEG₃-NH₂-GalCer 2a and 6-PEG₃-NHAc-GalCer 2b remains almost more potent to activate iNKTs than KRN7000 with 2 at 3 log of difference (Figure 11 G and H), while 6-PEG₃-NHCOBn-GalCer 2c have again the same profile effect than KRN7000.

These data indicates that the activity of PEG₃-NHR-GalCer derivatives is sensitive to the nature of NHR end terminal group displaying iNKT stimulation potency at a nearly pM range on transfected tumor cells and more interestingly at a sub nM range on non-CD1d-tumor cells when R is an acetate.

A comparison study was also run to evaluate the variation induces by the introduction of PEGₙ-maleimide (n=1 and 6) fragment at the end terminal position of the 6-PEG₃-NH₂-GalCer 2a (see Figure 12).

Data shows that 6-Mal-PEG₆-GalCer 3a presenting a maleimide activated function slightly decrease the activation of iNKT cells almost ten fold higher than KRN7000).

Regardless, these data already make the novel 6-Mal-PEG₆-GalCer 3a as candidate for an association with a therapeutic antibody or other biological carriers to induce cumulative biological anticancer cytokines release and cytotoxic effects of the carrier close to the tumor environment.

### Example 5: Elaboration of non-enzymatic cleavable GalCer/Cetuximab complexes

The synthesis of the 6-Mal-PEG₆-GalCer 3a from 6-PEG₃-NH₂-GalCer 2a was optimized. Intermediate 2a presents a long bait featuring a suitable reactive maleimide ending group (dashed line box) aimed to react *in situ* with Traut activated Cetuximab antibody to achieve the covalent linkage between the two partners (Schema 4).

It has also been successfully experimented the one pot process to access the GalCer/Cetuximab complex C1 from 6-PEG₃-NH₂-GalCer 2a without purification of the maleimide 6-Mal-PEG₆-Galcer intermediate 3a. For this purpose 6-PEG₃-NH₂-GalCer 2a was dissolved in phosphate buffer (PB) with 10% DMSO at 20°C and then directly reacted with Maleimide-PEGs-succinimide linker *(length of 6 PEG units was chosen in accordance with our previous results)* to give 6-Mal-PEG₆-Galcer 3a which was directly incubated with activated Traut cetuximab partner leading to the GalCer/Cetuximab complex C1.

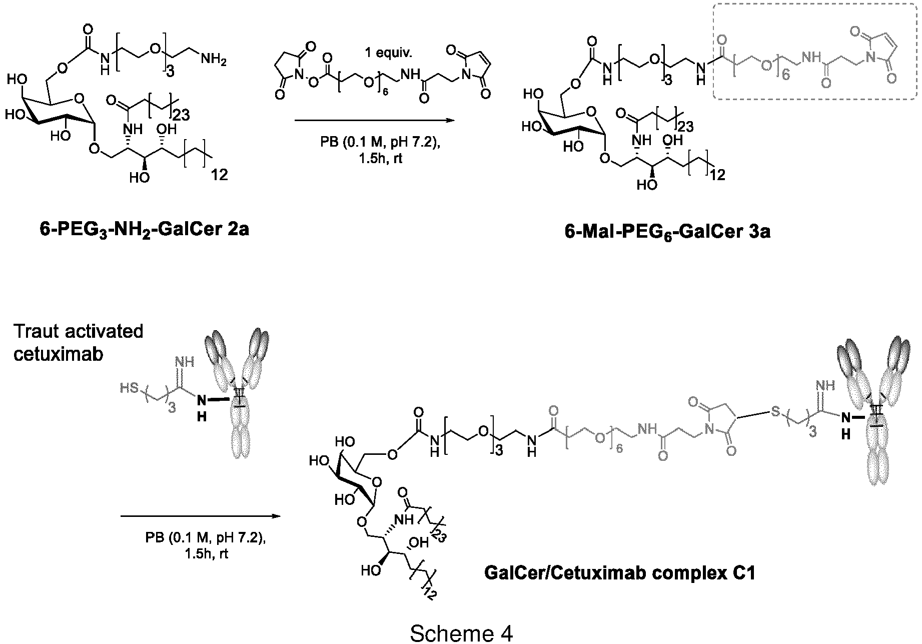

### (i) Coupling of glycolipids to cetuximab and purification

### a) Coupling conditions

The following conditions have been retained for coupling to cetuximab.

First cetuximab was modified by adding TRAUT functions, with a ratio of 100 TRAUT molecules for 1 antibody. It allowed addition of at least 4 TRAUT functions to cetuximab, as determined by the Ellman reaction previously investigated (data not schown). The mixture was washed and then 1/1 equivalent of 6-Mal-PEG₆-GalCer 3a and activated cetuximab were incubated to initiate the linkage.

**Table 7: Coupling conditions**

| **CONDITION** | **FRACTION B** | **FRACTION A** |
|---|---|---|
| | Cetuximab + 6-PEG₃-NH₂-GalCer **2a** | Modified Cetuximab with TRAUT + 6-Mal-PEG₆-GalCer **3a** |
| **TRAUT ratio** | No TRAUT | 1/100 |
| **Glycolipids Ratio** | 1/1 | 1/1 |
| **Coupling process** | No | Yes |
| **Washing process** | Yes | Yes |

The main difficulty encountered in these experiments is to ensure the elimination of the unreacted glycolipids in the medium after the coupling reaction with the antibody. Various methods were tested: Protein A, exclusion chromatography, electrophoresis, filtrations. Success was encountered when the resulting fractions were washed 3 time by filtration on VivaSpin15 coloumn (MWCO : 50Kda).

Two different experimental conditions were used to validate the purification process (see Table 7).
- **Fraction B:** Cetuximab is not modified by TRAUT activation and 6-PEG₃-NH₂-GalCer 2a, which does not have chemical ability to link to the cetuximab, was added. This condition was used as a control to follow the elimination of unbound glycolipid under washing conditions. Considering the high reactivity of 6-PEG₃-NH₂-GalCer 2a on iNKT stimulation, the presence of remaining derivatives, even in trace, would be detected by a significant cytokine release;
- **Fraction A:** use of 6-Mal-PEG₆-GalCer 3a and TRAUT activated Cetuximab to provide covalent linkage of glycolipid with the antibody and targeted GalCer/Cetuximab complex C1.

### b) Purification and activation of iNKT by GalCer/Cetuximab complex

Experiments were run on two series of transfected-CD1d Hela cells and non-CD1d-Hela Cells and 3 diluted samples of fractions A and B (10 µg/ml, 1 µg/ml and 0,1 µg/ml) were evaluated. In each series of Hela cells stimulation activity of the glycolipid alone was previously evaluated as reference following IFN-γ secretion.

### Starting concentration =

Maximum theoretical concentration of free glycolipid (unbounded) that can remain in the diluted fractions after coupling reaction if the washing process is inefficient.

### Observed concentration =

Estimated theoretical concentration of glycolipid that have to be remained in the diluted fractions after the washing process to induce the level of observed cytokine secretion (based on the reaction control with free glycolipid).

The experiments show that the washing process provides an elimination of the glycolipids (up to 99,9%).

Results with fraction B used as control experiment:
- As shown in Figure 14 A, left panel, after purification, the fraction resulting from the mixture of Cetuximab + 6-PEG₃-NH₂-GalCer 2a, which cannot link together, lead to a decrease of cytokine release following the 1/10^{th} dilutions. In this experiment using high sensitive CD1d-Hela cells, the concentration of the remaining 6-PEG₃-NH₂-GalCer 2a in the diluted fraction samples (observed concentration) appears at least to be 3 log lower than the starting concentration added in the mixture (Starting concentration). At a dilution of 0,1 µg/ml its activity become negligible leading to a lack of iNKT stimulation.
- Same experiment using non-CD1d Hela cells (Figure 14 B, left panel) leads to the lack of cytokine release even at 10 µg/ml, indicating that in physiological model, unbounded 6-PEG₃-NH₂-GalCer 2a can be considered totally removed from the fraction B by the washing process, or at least as trace (<10⁻¹⁰M limit for detection on non transfected HeLa cells).

Results with fraction A = linked Cetuximab-GalCer complex C1 :
As shown, the complex GalCer/Cetuximab C1 formed after TRAUT activation of the antibody in the presence of 6-Mal-PEG₆-GalCer 3a induces IFN-γ cytokine release by iNKT cells from all diluted fractions (10 µg/ml, 1 µg/ml and 0,1 µg/ml). These surprising results were observed from both high sensitive transfected-CD1d cells and also from non-CD1d-cells (Figures 15 C, left panel and 15 D, left panel, respectively).
The main interesting think is that iNKT activation is maintained despite the dilutions of the fractions in both cases. These results seems to indicate the activation of a pool of CD1d that could be surexpressed in the presence of Cetuximab-GalCer complex C1.

It should be kept in mind that starting concentration is the maximum theoretical concentrations estimated in the experiments are calculated in the case of a washing failure with no elimination of the Glycoplipd residues. However, it was known from previous experiences that almost 99.9% of glycolipids excess (unbounded) are eliminated after the first washing step. Thus, The theoretical concentrations estimated as reference after 3 washing steps are consequently largely overestimated and the "real" effect of the coupled Cetuximab-GalCer complex is thereby much more efficient in reality.

This latter experiment carried out from non-CD1d tumor cells indicates that coupled Cetuximab-GalCer complex C1 is able to induce a cytokine secretion **at an upper level** than the maximum release corresponding to a secretion induced by the 6-Mal-PEG₃-GalCer 3a used in its free form at an upper concentration.

These observations have been confirmed form non-CD1d HEK293 cells and PBMC cells (Peripheral Blood Mononuclear Cells) (*data not shown*).

### c) Mass spectroscopy of GalCer/Cetuximab C1

GalCer/Cetuximab C1 was analysed by mass spectrometry (ESI) to show that at least one or two molecules of 6-Mal-PEG₆-GalCer 3a analogue were linked to Cetuximab (see Table 8 below).

**Table 8: heavy chain of complex C1 with 1 and 2 GalCer moieties linked**

| | Average molar mass calculated by deconvolution of the spectrum (Da) | | | Retention time LC (min) | | |
|---|---|---|---|---|---|---|
| | Full antibody | Heavy chain | Light chain | Full antibody | Heavy chain | Light chain |
| Ab sample 004 | 153191 | 53005 | 23426 | 3,89 | 3,85-3,89 | 3,75 |
| | 155884 | 54220 | 23510 | | | |
| | | 56160 | 23594 | | | |
| Ab sample 001 | 152481 | 52757 | 23425 | 3,84 | 3,81-3,87 | 3,7 |
| Δ | 710 | 56160 | 23594 | / | / | / |
| | 3403 | | | | | |

Full antibody spectrum showed several major pics at 153191 Da et 155884 Da and deconvolution spectrum of pic at à 3,85 et 3,89 minutes of LC corresponds to the heavy chain of cetuximab linked to GalCer fragments (data not shown). The technique used for analysis was destructive for the antibody (leading to clear data only for linkage on heavy chain and unclear on light chain which is degraded), the maximum exact number of linked GalCer residues on the whole antibody cannot be not yet fully established.

### (ii) Coupling of glycolipids to cetuximab and purification (compared to coupling in the absence of TRAUT)

### a) Coupling conditions

The following conditions have been retained for coupling to cetuximab.

First cetuximab was modified by adding TRAUT functions, with a ratio of 100 TRAUT molecules for 1 antibody. It allowed addition of at least 4 TRAUT functions to cetuximab, as determined by the Ellman reaction previously investigated (data not shown). The mixture was washed and then 1/2 equivalent of 6-Mal-PEG₆-GalCer 3a and activated cetuximab were incubated to initiate the linkage.

**Table 9: Coupling conditions**

| **CONDITION** | **FRACTION B** | **FRACTION A** |
|---|---|---|
| | Cetuximab + 6-Mal-PEG₆-GalCer **3a** | Modified Cetuximab with TRAUT + 6-Mal-PEG₆-GalCer **3a** |
| **TRAUT ratio** | No TRAUT | 1/100 |
| **Glycolipids Ratio** | 1/2 | 1/2 |
| **Coupling process** | Yes | Yes |
| **Washing process** | Yes | Yes |

The main difficulty encountered in these experiments is to ensure the elimination of the unreacted glycolipids in the medium after the coupling reaction with the antibody. Various methods were tested: Protein A, exclusion chromatography, electrophoresis, filtrations. Success was encountered when the resulting fractions were washed 3 time by filtration on VivaSpin15 column (MWCO : 50Kda) follow by purification on Superdex 200 10/300 GL column for size exclusion chromatography.

Two different experimental conditions were used to validate the purification process (see Table 9).
- **Fraction B:** Cetuximab is not modified by TRAUT activation and 6-Mal-PEG₆-GalCer 3a. This condition was used as a control to follow the elimination of unbound glycolipid under washing conditions. Considering the high reactivity of 6-Mal-PEG₆-GalCer 3a on iNKT stimulation, the presence of remaining derivatives, even in trace, would be detected by a significant cytokine release;
- **Fraction A:** use of 6-Mal-PEG₆-GalCer 3a and TRAUT activated Cetuximab to provide covalent linkage of glycolipid with the antibody and targeted GalCer/Cetuximab complex C1.

### b) Mass spectroscopy of GalCer/Cetuximab C1

GalCer/Cetuximab C1 was analysed by mass spectrometry (ESI) to show two molecules of 6-Mal-PEG₆-GalCer 3a analogue were linked to Cetuximab (see Table 10 below).

**Table 10: heavy chain with 1 and 2 GalCer moieties linked**

| | | **Cetuximab Alone** | **Fraction B** | **Fraction A** |
|---|---|---|---|---|
| **Molecular Weight (Da)** | Full antibody | 152 583 | 152 533 | 156 065 |
| | Heavy chain | 52 904 | 52 904 | 53 232 + 55 541 |
| | Light chain | 23 426 | 23 427 | 23 604 |
| **Delta from Cetuximab Alone (Da)** | Full antibody | / | -50 | 3 482 |
| | Heavy chain | / | 0 | 328 + 2 637 |
| | Light chain | / | 1 | 178 |
| **N Coupling** | Full antibody | / | 0 | **2,2** |
| | Heavy chain | / | 0 | **0,2 + 1,7** |
| | Light chain | / | 0 | **0,1** |

Full antibody spectrum showed several major pics at 152 583 Da alone et 156 056 Da when coupling. This results indicate on average two molecules of 6-Mal-PEG₆-GalCer 3a (MW : 1 562 Da) are linked to the antibody. More precise study of heavy and light chains indicate that coupling mainly takes place on the heavy chain.

### c) Activation of iNKT by GalCer/Cetuximab complex

Experiments were run on non-CD1d-Hela Cells and 3 diluted samples of fractions A and B (10 µg/ml, 1 µg/ml and 0,1 µg/ml) were evaluated. Mass spectrometry analysis indicate that two molecules of 6-Mal-PEG₆-GalCer 3a were linked per antibody, so when 10pg/ml of Fraction A was used, it corresponds approximately to a concentration of 10⁻⁷M of equivalent 6-Mal-PEG₆-GalCer 3a. In each series of Hela cells stimulation activity of the glycolipid alone was previously evaluated as reference following IFN-γ secretion.

Results with fraction B used as control experiment:
As shown in Figure 16, after purification, the fraction resulting from the mixture of Cetuximab + 6-Mal-PEG₆-GalCer 3a, where no linkage was observed by mass spectrometry, no cytokine secretion was observed, indicating that washing process provide an elimination of unbounded glycolipids.

Results with fraction A = linked Cetuximab-GalCer complex C1 :
As shown, the complex GalCer/Cetuximab C1 formed after TRAUT activation of the antibody in the presence of 6-Mal-PEG₆-GalCer 3a induces IFN-γ cytokine release by iNKT cells from all diluted fractions (10 µg/ml, 1 µg/ml and 0,1 µg/ml). These surprising results were observed on non-CD1d-cells.
These results seems to indicate that Cetuximab-GalCer complex C1 is able to vectorize and release 6-Mal-PEG₆-GalCer 3a into the tumour cells allowing its presentation on the weak CD1d expression observed in HeLa cells leading to activation of iNKT cells.

### (iv) Cetuximab in GalCer/Cetuximab complexes is still able to recognize EGFR

It was then assessed if after coupling with 6-Mal-PEG₆-Galcer 3a, cetuximab was not altered and was still able to recognize EGFR. As shown in Figure 13, cetuximab binding to cells expressing EGFR was not altered after coupling since there was no difference between native cetuximab (direct from the bottle) and the 3 conditions tested for coupling reactions. Results were confirmed on 3 different cells lines that also express EGFR (not shown).

### (v) Cetuximab in GalCer/Cetuximab complexes and internalization

After EGFR recognition, Cetuximab is internalized, in clinical condition it allows to decrease surface expression of EGFR and reduces capacity of tumor cell to proliferate anarchically. Internalization of GalCer/Cetuximab complex C1 was followed by live microscopy for 21H. Complex C1 was labeled with a marker that only bright in red when it is internalized in acid endosome/lysosome. Most of HeLa cells were shown to have internalized complex C1 from 11H of co-incubation (data not shown). It was hypothesized that after internalization and support in lysosome, C1 complex release 6-Mal-PEG₆-GalCer 3a under acid conditions, allowing loading on CD1d molecule and presentation to the surface of tumour cells, leading to the iNKT cells activation observed above in part iii).

### (vi) ADCC behaviour of GalCer/Cetuximab complex

Another important function of cetuximab is its ability to induce ADCC. It was important to check the preservation of that behaviour from GalCer/Cetuximab complex C1. ADCC assays were performed on Hela cells and HCT116 cell line (Figure 14A and B respectively) because the latter proved to be the most responsive cell line among several cell lines that we tested in preliminary experiments and because HCT116 presents a Kras mutation. Target cells (HCT116 and HeLa cells) were coincubated for 1 hour with cetuximab alone (blue left panel) or Cetuximab-GalCer Complex C1 (red righ panel). Then ADCC was initiated by addition of NK92-CD16+ cells (a conventionally cell line used for ADCC assay) follow by an incubation for 24 hours. Mortality of target cells was analysed by Flow Cytometry.

ADCC potency of Cetuximab is preserved in all conditions on both models showing that linkage of glycolipid fragment does not alter the behaviour of the antibody with respect to EGFR recognition.

### (vii) Conclusion

GalCer/Cetuximab complex C1 with a covalent linkage are able to activate hiNKT cells to release cytokines using CD1d-tumor cells as antigen presenting partner. The important information is that carcinoma cells, previously considered as CD1d negative, can act as self-presenting cells through a very low pool of previously undetected CD1d, this behaviour being related to the exceptional potency of Cetuximab-GalCer complex C1. Additionally, GalCer derivative remains active despite its linkage to the antibody through a relatively short PEG spacer.

One hypothesis that can be made taking into account our data, is that Cetuximab achieves the vectorization of Cetuximab-GalCer complex C1 and the concentration of the glycolipids into the EGFR-tumor cells, probably through an internalization process. A subsequent release of glycolipids from the complex could then occur, maybe under acidic intracellular condition or antibody degradation, to allow its loading on an unknown internal pool of CD1d that can be consequently expressed at the surface of the tumor cell membrane. This CD1d-turnover appears to be able to mobilize iNKT cells and to induce cytokine stimulation restoring the immune response close to the tumor environment.

In that sense, Cetuximab-GalCer complex C1 can be regarded as potent drug candidate for immunotherapy providing enhance cytotoxic effect combining ADCC property of Cetuximab, or at least its cytotoxic effect, and a strong immunostimulation activity from iNKT.

### Conclusion

The present application discloses a new potent immunostimulator 6-PEG₃-NH₂-GalCer 2a expressing an hiNKT stimulation potency nearly 1000 fold higher than that of parent KRN7000.

More interestingly, corresponding GalCer/Cetuximab complex C1 (see Figure 15) keeps the ability to induce cytokines release by activating iNKT cells.

These data will lead to redefine the notion of CD1d negative cells since some tumoral cells, previously considered as CD1d negative (HeLa and HEK293), constitutively express CD1d at a sufficient level, albeit nearly undetectable, to effectively activate iNKT cells. iNKTs, themselves at low level in the experiment physiological level, when loaded with 6-PEG₃-NH₂-GalCer glycolipid 2a, induce cytokine release but as well when loaded with GalCer/Cetuximab complex C1. This suggests that the antibody can be already regarded as a good vehicle to carry the GalCer immunostimulator within the tumoral environment in order to initiate iNKT stimulation. This process can bypass the requirement for classical CD1d presenting cells (such as monocytes, macrophages or dendritic cells), cancer cells being able to self-present even when the glycolipid agonist is linked to the antibody.

## Claims

1. An iNKT stimulator, wherein said iNKT stimulator consists of a compound of the following formula (II): 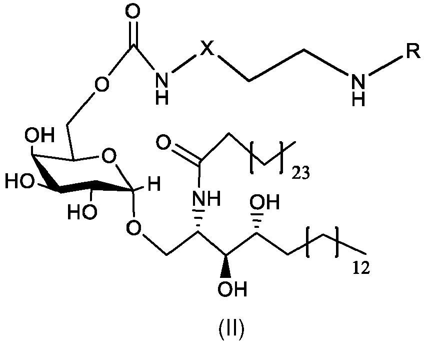
wherein X represents a PEG fragment -[CH₂-CH₂-O]ₘ with m being an integer from 1 to 24, an alkyl chain -(CH₂)ₙ- with n being an integer from 1 to 24 or a branched hydrocarbon group,
wherein R represents -CO-R1, -CONR1, -COOR1, -CSR1, -CSNR1 or -CSOR1, wherein R1 represents an alkyl, an aryl, an heterocyclic group, a PEG fragment - [CH₂-CH₂-O]ₚ-R2 with p being an integer from 1 to 24 or an alkyl chain -(CH₂)_{q}-R2 with q being an integer from 1 to 24,
wherein R2 represents a functional reacting group allowing coupling to a carrier D or represents L-D, wherein L is a linker and D is a carrier.

2. The iNKT stimulator according to claim 1, wherein said iNKT stimulator is selected from the group consisting of:
- a compound of the following formula (V): 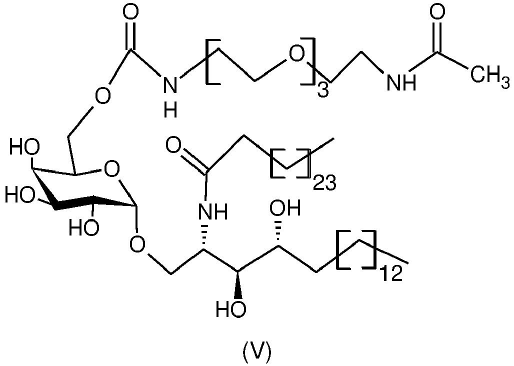
- a compound of the following formula (VI): 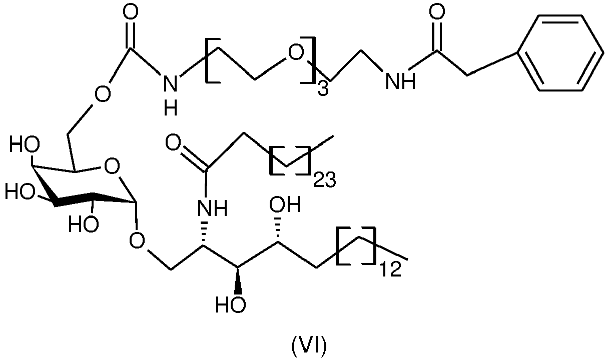
- a compound of the following formula (VII): 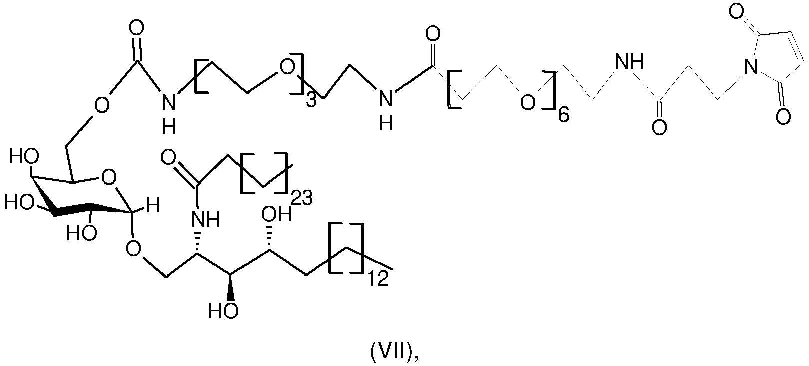 and
- a compound of the following formula (VIII):

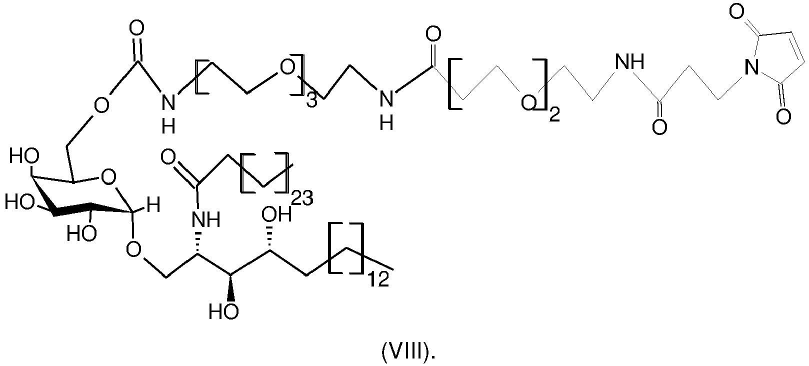

3. A conjugate comprising at least one iNKT stimulator coupled to at least one carrier, wherein said conjugate:
(i) comprises a compound of the formula (II) 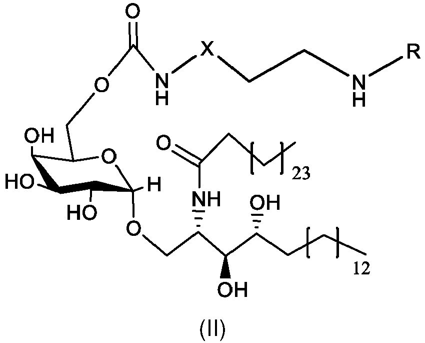 wherein X represents a PEG fragment -[CH2-CH2-O]ₘ with m being an integer from 1 to 24, an alkyl chain -(CH2)ₙ- with n being an integer from 1 to 24 or a branched hydrocarbon group, wherein R represents -CO-R1, -CONR1, -COOR1, -CSR1, -CSNR1 or -CSOR1 and wherein R1 represents a PEG fragment -[CH₂-CH₂-O]ₚ-L-D with p being an integer from 1 to 24 or an alkyl chain -(CH₂)_{q}-L-D with q being an integer from 1 to 24, wherein L is a linker and D is a carrier, or
(ii) is obtained by coupling to one carrier D at least one iNKT stimulator of the above formula (II), wherein X represents a PEG fragment -[CH2-CH2-O]ₘ with m being an integer from 1 to 24, an alkyl chain -(CH2)ₙ- with n being an integer from 1 to 24 or a branched hydrocarbon group, wherein R represents -CO-R1, - CONR1, -COOR1, -CSR1, -CSNR1 or -CSOR1, wherein R1 represents a PEG fragment -[CH2-CH2-O]ₚ-R2 with p being an integer from 1 to 24 or an alkyl chain -(CH2)_{q}-R2 with q being an integer from 1 to 24 and wherein R2 represents a functional reacting group allowing coupling to said carrier D.

4. The iNKT stimulator according to claim 1 or the conjugate according to claim 3, wherein said carrier D is a therapeutic agent and/or a targeting agent.

5. The iNKT stimulator according to claim 1 or 4 or the conjugate according to 3 or 4, wherein said carriers D is an antibody, antibody fragment, sugar, lectin, affitin, growth factor, antigen, a protein, peptide, glycoprotein, aptamer, loaded cell, virus and/or carbohydrate.

6. A vector comprising at least one iNKT stimulator according to any one of claims 1, 2, 4 or 5, at least one conjugate according to any one of claims 3 to 5 and/or at least one iNKT stimulator consisting of a compound of the following formula (IV)

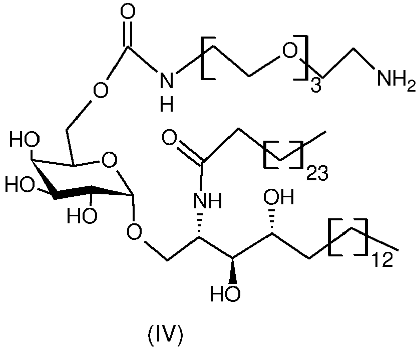

7. The vector according to claim 6, wherein said vector is a nanoparticle.

8. A pharmaceutical composition comprising:
(i) at least one iNKT stimulator according to any one of claims 1, 2, 4 and 5, at least one conjugate according to any one of claims 3 to 5, at least one vector according to 6 or 7 and/or at least one iNKT stimulator consisting of a compound of the following formula (IV): 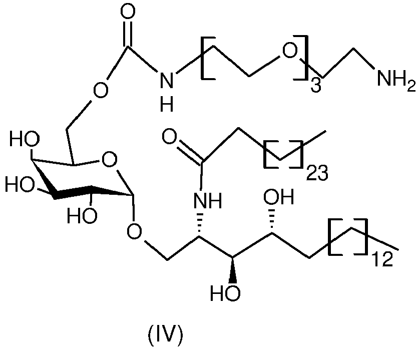 and
(ii) at least one pharmaceutically acceptable vehicle.

9. The iNKT stimulator according to any one of claims 1, 2, 4 and 5, the conjugate according to any one of claims 3 to 5, the vector according to claim 6 or 7, the pharmaceutical composition according to claim 8 or an iNKT stimulator consisting of a compound of the following formula (IV): 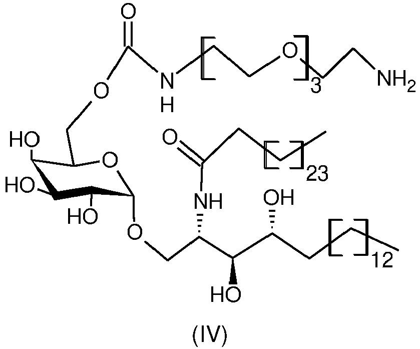 for use as a medicament.

10. The iNKT stimulator, the conjugate, the vector or the pharmaceutical composition for use according to claim 9, in the prevention and/or treatment of cancer, an autoimmune disease, an inflammatory disease, a viral infection, a bacterial infection and/or a parasitic disease.

11. An *in vitro* use of the iNKT stimulator according to any one of claims 1, 2, 4 or 5, of the conjugate according to any one of claims 3 to 5, of the vector according to claim 6 or 7 or of an iNKT stimulator consisting of a compound of the following formula (IV) 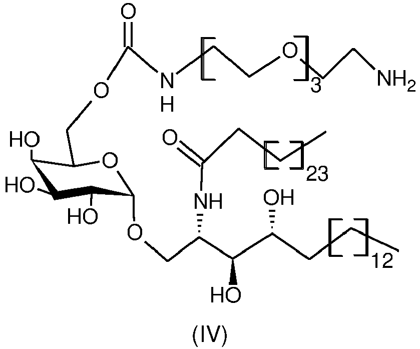 to activate iNKT cells.

## Patentansprüche

1. INKT-Stimulator, wobei der iNKT-Stimulator aus einer Verbindung der folgenden Formel (II) besteht: 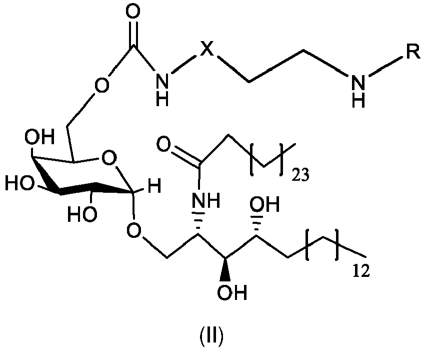
wobei X ein PEG-Fragment -[CH₂-CH₂-O]m, wobei m eine ganze Zahl von 1 bis 24 ist, eine Alkylkette -(CH₂)ₙ-, wobei n eine ganze Zahl von 1 bis 24 ist, oder eine verzweigte Kohlenwasserstoffgruppe darstellt,
wobei R -CO-R1, -CONR1, -COOR1, -CSR1, -CSNR1 oder -CSOR1 darstellt, wobei R1 eine Alkyl-, eine Aryl-, eine heterocyclische Gruppe, ein PEG-Fragment -[CH₂-CH₂-O]ₚ-R2, wobei p eine ganze Zahl von 1 bis 24 ist, oder eine Alkylkette -(CH₂)_{q}-R2, wobei q eine ganze Zahl von 1 bis 24 ist, darstellt,
wobei R2 eine funktionelle Reaktionsgruppe darstellt, die eine Kopplung an einen Träger D ermöglicht, oder L-D darstellt, wobei L ein Linker und D ein Träger ist.

2. INKT-Stimulator nach Anspruch 1, wobei der iNKT-Stimulator ausgewählt ist aus der Gruppe, bestehend aus:
- einer Verbindung der folgenden Formel (V): 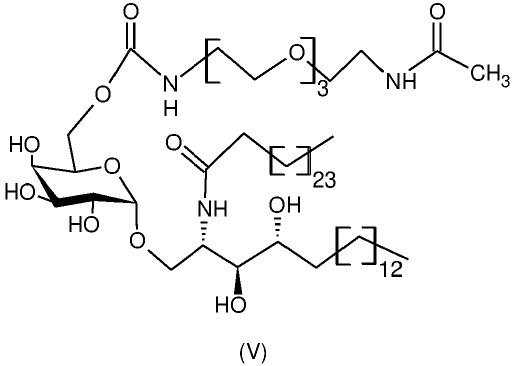
- einer Verbindung der folgenden Formel (VI): 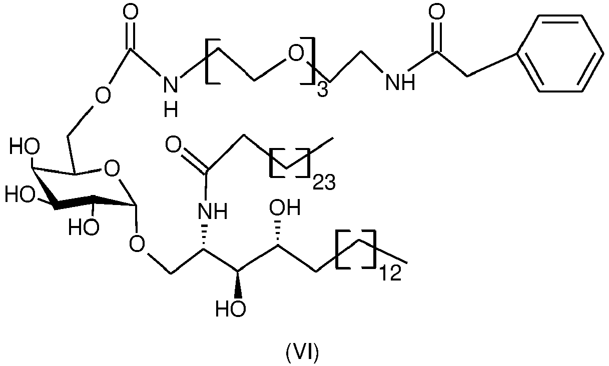
- einer Verbindung der folgenden Formel (VII): 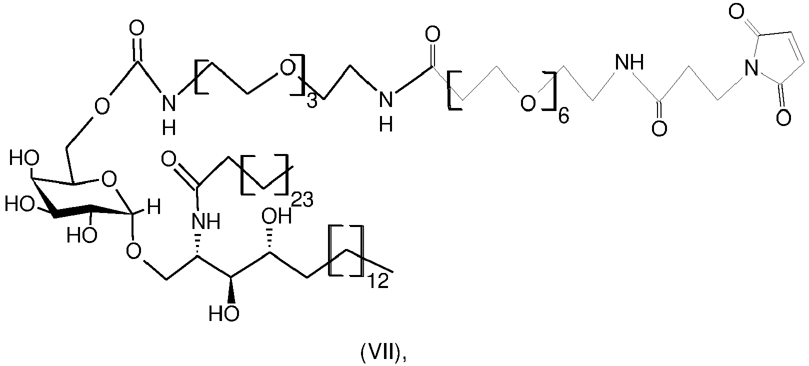 und
- einer Verbindung der folgenden Formel (VIII):

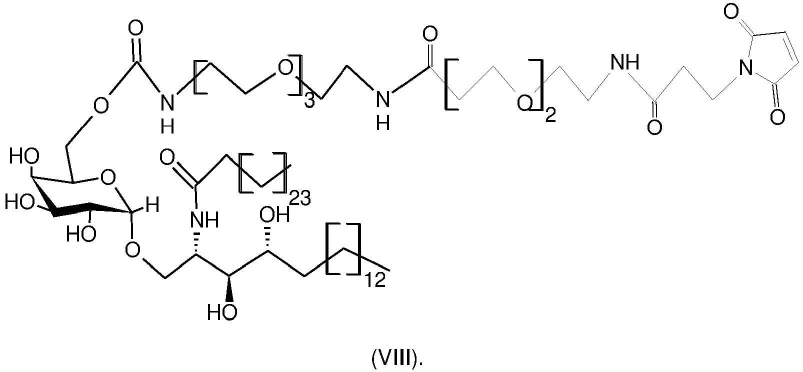

3. Konjugat, umfassend mindestens einen iNKT-Stimulator, der an mindestens einen Träger gekoppelt ist, wobei das Konjugat:
(i) eine Verbindung der Formel (II) umfasst 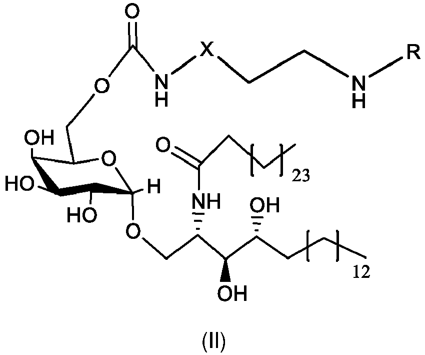 wobei X ein PEG-Fragment -[CH2-CH2-O]ₘ, wobei m eine ganze Zahl von 1 bis 24 ist, eine Alkylkette -(CH2)ₙ-, wobei n eine ganze Zahl von 1 bis 24 ist, oder eine verzweigte Kohlenwasserstoffgruppe darstellt, wobei R -CO-R1, -CONR1, -COOR1, -CSR1, - CSNR1 oder -CSOR1 darstellt, und worin R1 ein PEG-Fragment -[CH₂- CH₂-O]ₚ-L-D, wobei p eine ganze Zahl von 1 bis 24 ist, oder eine Alkylkette -(CH₂)_{q}-L-D, wobei q eine ganze Zahl von 1 bis 24 ist, darstellt, worin L ein Linker ist und D ein Träger ist, oder
(ii) durch Koppeln von mindestens einem iNKT-Stimulator der obigen Formel (II) an einen Träger D erlangt wird, wobei X ein PEG-Fragment -[CH2-CH2-O]ₘ, wobei m eine ganze Zahl von 1 bis 24 ist, eine Alkylkette -(CH2)ₙ-, wobei n eine ganze Zahl von 1 bis 24 ist, oder eine verzweigte Kohlenwasserstoffgruppe darstellt, wobei R -CO-R1, - CONR1, -COOR1, -CSR1, -CSNR1 oder -CSOR1 darstellt, und wobei R1 a PEG-Fragment -[CH2-CH2-O]ₚ-R2, wobei p eine ganze Zahl von 1 bis 24 ist, oder eine Alkylkette -(CH2)_{q}-R2, wobei q eine ganze Zahl von 1 bis 24 ist, darstellt, und wobei R2 eine funktionelle Reaktionsgruppe darstellt, die eine Kopplung an den Träger D ermöglicht.

4. iNKT-Stimulator nach Anspruch 1 oder Konjugat nach Anspruch 3, wobei der Träger D ein therapeutisches Mittel und/oder ein Targeting-Mittel ist.

5. iNKT-Stimulator nach Anspruch 1 oder 4 oder Konjugat nach 3 oder 4, wobei der Träger D ein Antikörper, ein Antikörperfragment, ein Zucker, ein Lektin, ein Affitin, ein Wachstumsfaktor, ein Antigen, ein Protein, ein Peptid, ein Glykoprotein, ein Aptamer, eine geladene Zelle, ein Virus und/oder ein Kohlenhydrat ist.

6. Vektor, umfassend mindestens einen iNKT-Stimulator nach einem der Ansprüche 1, 2, 4 oder 5, mindestens ein Konjugat nach einem der Ansprüche 3 bis 5 und/oder mindestens einen iNKT-Stimulator, bestehend aus einer Verbindung der folgenden Formel (IV)

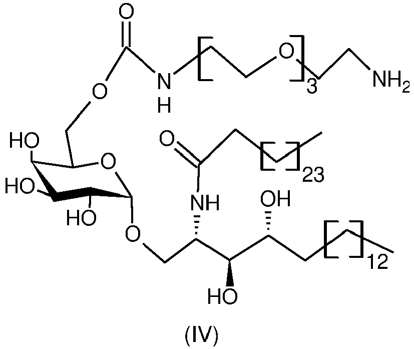

7. Verbindung nach Anspruch 6, wobei der Vektor ein Nanopartikel ist.

8. Pharmazeutische Zusammensetzung, umfassend:
(i) mindestens einen iNKT-Stimulator nach einem der Ansprüche 1, 2, 4 und 5, mindestens ein Konjugat nach einem der Ansprüche 3 bis 5, mindestens einen Vektor nach 6 oder 7 und/oder mindestens einen iNKT-Stimulator bestehend aus einer Verbindung der folgenden Formel (IV): 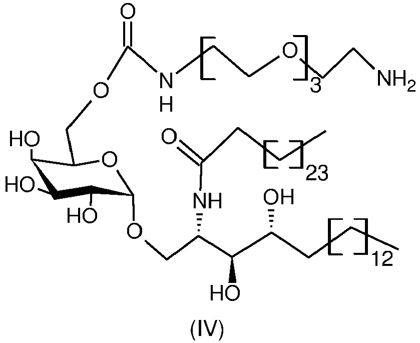 und
(ii) mindestens ein pharmazeutisch annehmbares Vehikel.

9. iNKT-Stimulator nach einem der Ansprüche 1, 2, 4 und 5, Konjugat nach einem der Ansprüche 3 bis 5, Vektor nach Anspruch 6 oder 7, pharmazeutische Zusammensetzung nach Anspruch 8 oder iNKT-Stimulator bestehend aus einer Verbindung der folgenden Formel (IV): 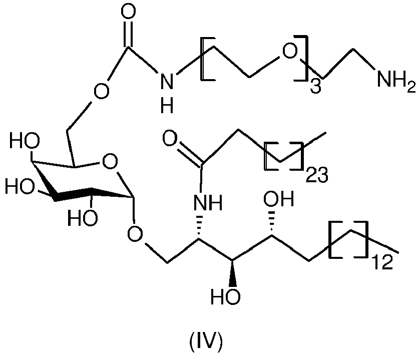 zur Verwendung als Medikament.

10. iNKT-Stimulator, Konjugat, Vektor oder pharmazeutische Zusammensetzung zur Verwendung nach Anspruch 9 bei der Vorbeugung und/oder Behandlung von Krebs, einer Autoimmunerkrankung, einer entzündlichen Erkrankung, einer viralen Infektion, einer bakteriellen Infektion und/oder einer parasitären Erkrankung.

11. *In-vitro-Verwendung* des iNKT-Stimulators nach einem der Ansprüche 1, 2, 4 oder 5, des Konjugats nach einem der Ansprüche 3 bis 5, des Vektors nach Anspruch 6 oder 7 oder eines iNKT-Stimulators, bestehend aus einer Verbindung der folgenden Formel (IV) 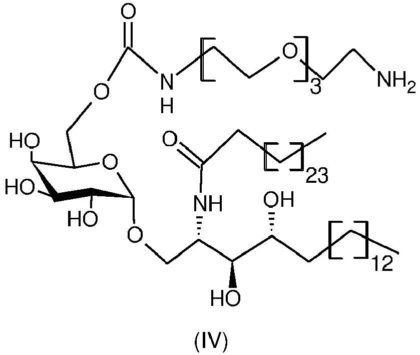 um iNKT-Zellen zu aktivieren.

## Revendications

1. Stimulateur iNKT, dans lequel ledit stimulateur iNKT consiste en un composé de la formule (II) suivante : 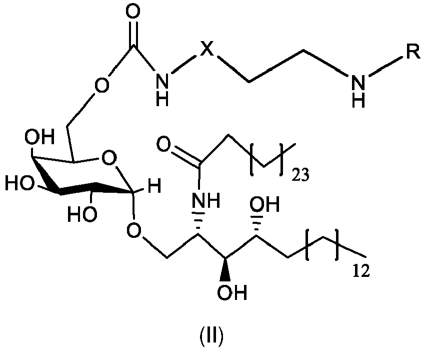
dans lequel X représente un fragment PEG-[CH₂-CH₂-O]ₘ m étant un nombre entier de 1 à 24, une chaîne alkyle -(CH₂)ₙ- n étant un nombre entier de 1 à 24, ou un groupe hydrocarboné ramifié,
dans lequel R représente -CO-R1, -CONR1, -COOR1, -CSR1, -CSNR1 ou -CSOR1, où R1 représente un alkyle, un aryle, un groupe hétérocyclique, un fragment PEG - [CH₂-CH₂-O]ₚ-R2, p étant un nombre entier de 1 à 24, ou une chaîne alkyle -(CH₂)_{q}-R2, q étant un nombre entier de 1 à 24,
dans lequel R2 représente un groupe fonctionnel réactif permettant le couplage à un support D ou représente L-D, dans lequel L est un agent de liaison et D est un support.

2. Stimulateur iNKT selon la revendication 1, dans lequel ledit stimulateur iNKT est choisi dans le groupe constitué par :
- un composé de la formule (V) suivante : 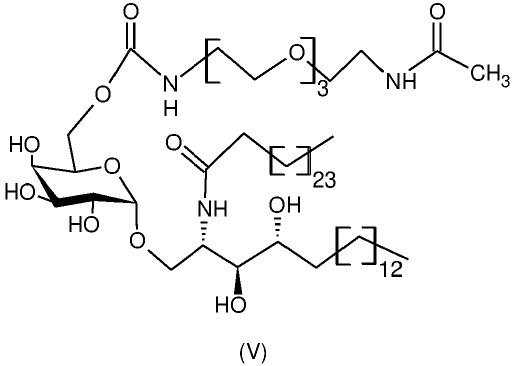
- un composé de la formule (VI) suivante : 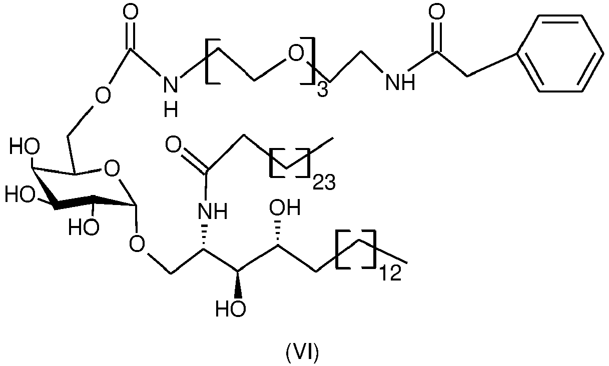
- un composé de la formule (VII) suivante : 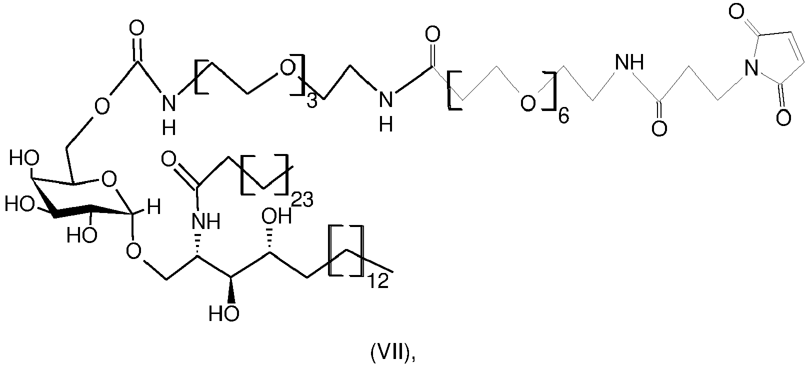 et
- un composé de la formule (VIII) suivante :

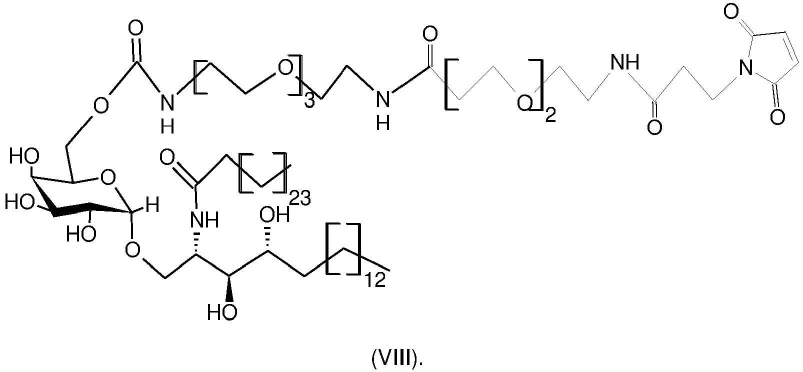

3. Conjugué comprenant au moins un stimulateur iNKT couplé à au moins un support, dans lequel ledit conjugué :
(i) comprend un composé de la formule (II) 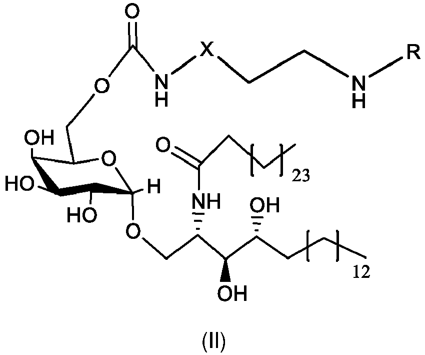 dans lequel X représente un fragment PEG -[CH2-CH2-O]ₘ, m étant un nombre entier de 1 à 24, une chaîne alkyle -(CH2)ₙ-, n étant un nombre entier de 1 à 24, ou un groupe hydrocarboné ramifié, dans lequel R représente -CO-R1, -CONR1, -COOR1, -CSR1, - CSNR1 ou -CSOR1 et dans lequel R1 représente un fragment PEG -[CH₂-CH₂-O]ₚ-L-D, p étant un nombre entier de 1 à 24 ou une chaîne alkyle -(CH₂)_{q}-L-D, q étant un nombre entier de 1 à 24, dans lequel L est un agent de liaison et D est un support, ou
(ii) est obtenu par couplage à un support D d'au moins un stimulateur iNKT de la formule (II) ci-dessus, dans lequel X représente un fragment PEG -[CH2-CH2-O]ₘ, m étant un nombre entier de 1 à 24, une chaîne alkyle -(CH2)ₙ-, n étant un nombre entier de 1 à 24, ou un groupe hydrocarboné ramifié, dans lequel R représente -CO-R1, - CONR1, -COOR1, -CSR1, -CSNR1 ou -CSOR1, dans lequel R1 représente un fragment PEG -[CH2-CH2-O]ₚ-R2, p étant un nombre entier de 1 à 24 ou une chaîne alkyle -(CH2)_{q}-R2, q étant un nombre entier de 1 à 24 et dans lequel R2 représente un groupe fonctionnel réactif permettant le couplage audit support D.

4. Stimulateur iNKT selon la revendication 1 ou conjugué selon la revendication 3, dans lequel le support D est un agent thérapeutique et/ou un agent de ciblage.

5. Stimulateur iNKT selon la revendication 1 ou 4 ou conjugué selon la revendication 3 ou 4, dans lequel ledit transporteur D est un anticorps, un fragment d'anticorps, un sucre, une lectine, une affitine, un facteur de croissance, un antigène, une protéine, un peptide, une glycoprotéine, un aptamère, une cellule chargée, un virus et/ou un hydrate de carbone.

6. Vecteur comprenant au moins un stimulateur iNKT selon l'une quelconque des revendications 1, 2, 4 ou 5, au moins un conjugué selon l'une quelconque des revendications 3 à 5 et/ou au moins un stimulateur iNKT constitué par un composé de la formule (IV) suivante

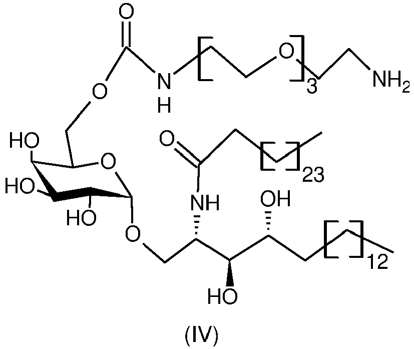

7. Vecteur selon la revendication 6, dans lequel ledit vecteur est une nanoparticule.

8. Composition pharmaceutique comprenant :
(i) au moins un stimulateur iNKT selon l'une quelconque des revendications 1, 2, 4 et 5, au moins un conjugué selon l'une quelconque des revendications 3 à 5, au moins un vecteur selon la revendication 6 ou 7 et/ou au moins un stimulateur iNKT constitué par un composé de la formule (IV) suivante : 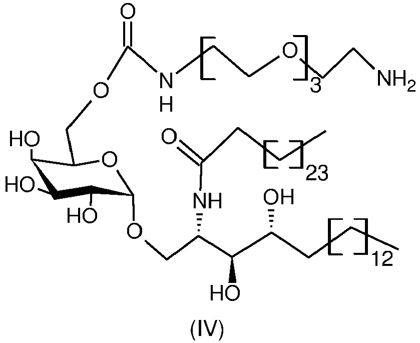 et
(ii) au moins un véhicule pharmaceutiquement acceptable.

9. Stimulateur iNKT selon l'une quelconque des revendications 1, 2, 4 et 5, conjugué selon l'une quelconque des revendications 3 à 5, vecteur selon la revendication 6 ou 7, composition pharmaceutique selon la revendication 8 ou stimulateur iNKT constitué par un composé de la formule (IV) suivante : 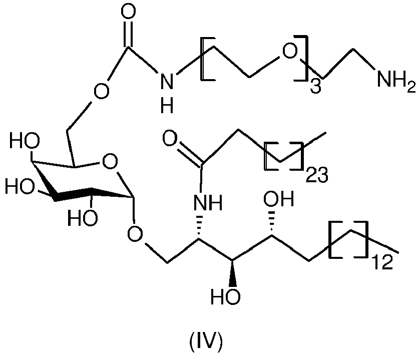 pour une utilisation en tant que médicament.

10. Stimulateur iNKT, conjugué, vecteur ou composition pharmaceutique pour une utilisation selon la revendication 9, dans la prévention et/ou le traitement du cancer, d'une maladie auto-immune, d'une maladie inflammatoire, d'une infection virale, d'une infection bactérienne et/ou d'une maladie parasitaire.

11. Utilisation *in vitro* du stimulateur iNKT selon l'une quelconque des revendications 1, 2, 4 ou 5, du conjugué selon l'une quelconque des revendications 3 à 5, du vecteur selon la revendication 6 ou 7 ou d'un stimulateur iNKT constitué par un composé de la formule (IV) suivante 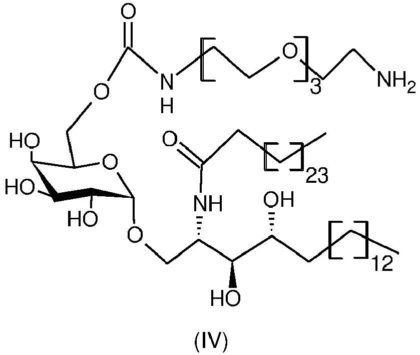 pour activer les cellules iNKT.
